# EUROPEAN PATENT SPECIFICATION

(11) **EP 2 081 960 B1**
(45) Date of publication and mention of the grant of the patent: **27.06.2018**
(21) Application number: 07821856.7
(22) Date of filing: 25.10.2007
(51) Int. Cl.: C07K 16/28, C07K 16/30, A61K 39/395, A61K 9/00

(54) **INTRANASAL DELIVERY OF POLYPEPTIDES AND PROTEINS**
INTRANASALE VERABREICHUNG VON POLYPEPTIDEN UND PROTEINEN
ADMINISTRATION INTRANASALE DE POLYPEPTIDES ET DE PROTÉINES

(30) Priority: 27.10.2006 US 855001 P; 31.10.2006 US 855544 P
(43) Date of publication of application: 29.07.2009
(73) Proprietor: Ablynx N.V., 9052 Ghent-Zwijnaarde (BE)
(72) Inventor: MERCHIERS, Pascal, Gérard, 2460 Tielen (BE); REVETS, Hilde, Adi, Pierrette, 1860 Meise (BE)
(74) Representative: Hoffmann Eitle
(86) International application number: PCT/EP2007/061493
(87) International publication number: WO 2008/049897

(56) References cited:
- WO-A-02/074243
- WO-A-2004/041867
- WO-A-2006/040153
- WO-A-2006/122825
- WO-A-2007/022416
- WO-A-2007/104529
- WO-A2-2004/041867
- WO-A2-2006/091332
- WO-A2-2007/049017
- US-A1- 2006 034 845
- US-A1- 2006 149 041
- BIE DE J J ET AL: "EFFECT OF INTERLEUKIN-16-BLOCKING PEPTIDE ON PARAMETERS OF ALLERGIC ASTHMA IN A MURINE MODEL" EUROPEAN JOURNAL OF PHARMACOLOGY, AMSTERDAM, NL, vol. 383, no. 2, 27 October 1999 (1999-10-27), pages 189-196, XP001154528 ISSN: 0014-2999
- OHTA ET AL: "Diesel exhaust particulate induces airway hyperresponsiveness in a murine model: Essential role of GM-CSF" JOURNAL OF ALLERGY AND CLINICAL IMMUNOLOGY, MOSBY - YEARLY BOOK, INC, US, vol. 104, no. 5, November 1999 (1999-11), pages 1024-1030, XP005687407 ISSN: 0091-6749
- FRENKEL DAN ET AL: "Filamentous phage as vector-mediated antibody delivery to the brain" PROCEEDINGS OF THE NATIONAL ACADEMY OF SCIENCES OF USA, NATIONAL ACADEMY OF SCIENCE, WASHINGTON, DC, US, vol. 99, no. 8, 16 April 2002 (2002-04-16), pages 5675-5679, XP002451994 ISSN: 0027-8424
- MURUGANANDAM A ET AL: "Selection of phage-displayed llama single-domain antibodies that transmigrate across human blood-brain barrier endothelium" FASEB JOURNAL (FEDERATION OF AMERICAN SOCIETIES FOR EXPERIMENTAL BIOLOGY), BETHESDA, US, vol. 16, no. 2, February 2002 (2002-02), pages 240-242, XP002310745 ISSN: 0892-6638
- GUIRAKHOO F ET AL: "Cloning, expression and functional activities of a single chain antibody fragment directed to fusion protein of respiratory syncytial virus", IMMUNOTECHNOLOGY, ELSEVIER SCIENCE PUBLISHERS BV, NL LNKD- DOI:10.1016/1380-2933(96)00053-X, vol. 2, no. 3, 1 September 1996 (1996-09-01), pages 219-228, XP004070296, ISSN: 1380-2933
- ZUANY-AMORIM C ET AL: "Interleukin-10 inhibits antigen-induced cellular recruitment into the airways of sensitized mice.", THE JOURNAL OF CLINICAL INVESTIGATION JUN 1995 LNKD- PUBMED:7769104, vol. 95, no. 6, June 1995 (1995-06), pages 2644-2651, ISSN: 0021-9738
- EIGENBROD T ET AL: "LOCAL DELIVERY OF AN ANTI-INTERLEUKIN-6 RECEPTOR ANTIBODY SUPPRESSES LUNG INFLAMMATION, BY INDUCTION OF INTRAPULMONARY CELL APOPTOSIS ON A MOUSE MODEL OF ASTHMA", JOURNAL OF ALLERGY AND CLINICAL IMMUNOLOGY, MOSBY, INC, US, vol. 107, no. 2, 1 February 2001 (2001-02-01), page S43, XP001069968, ISSN: 0091-6749
- H. ULRICHTS ET AL: "Antithrombotic drug candidate ALX-0081 shows superior preclinical efficacy and safety compared with currently marketed antiplatelet drugs", BLOOD, vol. 118, no. 3, 16 May 2011 (2011-05-16), pages 757-765, XP055109919, ISSN: 0006-4971, DOI: 10.1182/blood-2010-11-317859
- COPPIETERS KEN ET AL: "Formatted anti-tumor necrosis factor alpha VHH proteins derived from camelids show superior potency and targeting to inflamed joints in a murine model of collagen-induced arthritis", ARTHRITIS & RHEUMATISM, WILEY, US, vol. 54, no. 6, 1 June 2006 (2006-06-01), pages 1856-1866, XP002410952, ISSN: 0004-3591, DOI: 10.1002/ART.21827
- HOLT L J ET AL: "Domain antibodies: proteins for therapy", TRENDS IN BIOTECHNOLOGY, ELSEVIER PUBLICATIONS, CAMBRIDGE, GB, vol. 21, no. 11, 1 November 2003 (2003-11-01), pages 484-490, XP004467495, ISSN: 0167-7799, DOI: 10.1016/J.TIBTECH.2003.08.007
- ZHENG C J ET AL: "Therapeutic targets: progress of their exploration and investigation of their characteristics.", PHARMACOLOGICAL REVIEWS JUN 2006, vol. 58, no. 2, June 2006 (2006-06), pages 259-279, ISSN: 0031-6997
- BROWN LARRY R: "Commercial challenges of protein drug delivery.", EXPERT OPINION ON DRUG DELIVERY JAN 2005, vol. 2, no. 1, January 2005 (2005-01), pages 29-42, ISSN: 1742-5247

## Description

### FIELD OF THE INVENTION

The present invention relates to the intranasal delivery of polypeptides and proteins. More particularly, the present invention relates to the intranasal delivery of polypeptides and proteins comprising a Nanobody®, a domain antibody, a single domain antibody or a "dAb". *[Note: Nanobody*®*, Nanobodies*® *and Nanoclone*® *are trademarks of Ablynx N.V.]* The present invention relates to compositions and delivery devices suitable for intranasal delivery of said polypeptides and proteins. The invention also relates to methods for the treatment of a subject comprising the delivery of said polypeptides and proteins to said subject by the nasal route.

Other aspects, embodiments, advantages and applications of the invention will become clear from the further description herein.

### BACKGROUND ART

Administration of conventional low molecular weight drugs by non-invasive routes has been a well established practice. Therapeutic peptides and proteins, however, are often unstable, have large molecular weights and are polar in nature. These properties lead to poor permeability through biological membranes. When administered orally, for example, they are susceptible to proteolytic degradation in the gastrointestinal tracts and only pass with difficulty into the body fluids. For this reason, therapeutic peptides and proteins have hitherto been administered mostly by injection or infusion.

Injections, however, are always inconvenient and when administration is to be effected at regular intervals and for long term therapy, it can cause considerable pain and discomfort to the patient. The finding of viable alternative means of administration, causing less inconvenience to the patient and preferably allowing ready self-application while at the same time achieving sufficient efficacy for effective treatment would therefore be desirable. The development of a convenient, non-invasive, alternative route of administration for therapeutic peptides and proteins with comparable pharmacokinetic performance to parenteral injection would represent a significant advance in peptide and protein therapy.

Intranasal delivery is a needle-free, patient-friendly administration route. Because needles are not involved, this method of drug delivery is virtually painless. For patients who fear injections, intranasal delivery offers a more acceptable alternative. Additionally, the simplicity of nasal delivery would allow for self-administration in a home setting. This route is clearly of great advantage over parenteral administration which has generally to be given under medical supervision. Other advantages of intranasal delivery include the high permeability of the nasal epithelium and, as a result of the rather large surface area of the nasal cavity and the relatively high blood flow, rapid absorption. Compared to other non-injection administrations, such as oral formulation, nasal delivery is an attractive route for its characteristics of avoiding liver first-pass effect, rapid on set action and a higher bioavailability (Hussain, Adv. Drug Deliv. Rev. 1998 Jan 5; 29(1-2): 39-49; Illum, J. Control Release 2003 Feb 21; 87(1-3): 187-98).

However, the absorption of various larger peptides and proteins across the nasal mucosa is often insufficient to achieve sufficient amounts of these peptide in the bloodstream in order to provide an effective therapy. The first step in the absorption of peptides from the nasal cavity is passage through the mucus (Khanvilkar et al. Adv. Drug Deliv. Rev. 2001; 28: 173-193). Mucin, the principal protein in the mucus, has the potential to bind solutes, hindering diffusion. Additionally, structural changes in the mucus layer are possible as a result of environmental changes (i.e., pH, temperature, etc.). Subsequent to the passage through the mucus, there are several mechanisms for absorption through the mucosa (Marttin et al., Pharm. Res. 1997; 14: 631-637). These include transcellular or simple diffusion across the membrane, paracellular transport via movement between cells, and transcytosis by vesicle carriers (McMartin et al., J. Pharm. Sci. 1987; 76: 535-540). There are several proteolytic enzymes on the nasal mucosa surface, in paracellular and in the epithelium cells which could result in the degradation of therapeutic peptides and proteins (Sarkar, Pharm. Res. 1992; 9: 1-9; Yamamoto et al., Life Sci. 1990; 47: 2465). In addition to hindrances during their passage through the nasal mucosa, another obstacle to peptide and protein absorption is therefore their potential metabolism before reaching the systemic circulation and their limited survival in the cavity. In conclusion, bioavailability of therapeutic peptides and proteins after nasal administration tends to be relatively low due primarily to their large molecular size to enable easy passage and due to rapid enzyme degradation. As the number of amino acids increases beyond about 20, bioavailability becomes very low (Wearly, Crit. Rev. Ther. Drug Carrier Syst. 1991; 8: 331-394).

Accordingly, although nasal delivery possesses many advantages, delivery of therapeutic peptides and proteins through the nasal route is still much less explored and is considered less efficient than through injection, due mainly to intrinsic poor permeability and metabolism. Even so, some peptide products have successfully reached the market as intranasal formulations, albeit as simple formulations with relatively low bioavailability due to the permeation barrier presented by the nasal mucosa (Illum, The nasal route for delivery of polypeptides. In: Peptide and Protein Drug Delivery. Eds. Frokjaer, S., Christrup, L., Krogsgaard-Larsen, P., Munksgaard, Copenhagen 1990, p 157-170). FDA approved examples include DDAVP®, Miacalcin™ (calcitonin) and Synarel™ (nafarelin). Clinical trials have demonstrated that influenza (trivalent) and diphtheria toxin (CRM-197) can be delivered nasally with good effect (Davis, Adv. Drug Del. Rev. 2001; 51: 21-42). Larsen et al. (Eur. J. Clin. Pharmacol. 1987; 33: 155-159) concluded that intranasal application of the low molecular weight polypeptide buserelin represented a reliable mode of administration. Other examples of intranasal formulations of peptides such as insulin, human growth hormone, glucagon, hirudin, human interferon-β and human parathyroid hormone are discussed in Costantino et al. (www.ondrugdelivery.com, 2005; Sakr, Int. J. Pharmaceutics 1996, 132: 189-194; O'Hagan et al., Pharm. Res. 1990, 7: 772-776; Cefalu, Diabetes Care 2004, 27: 239-245; Zhang et al., Biol. Pharm. Bull 2005, 28: 2263-2267).

The present inventors have now found that a certain class of therapeutic polypeptides or proteins, i.e. polypeptides and proteins comprising a Nanobody, a domain antibody, a single domain antibody or "dAb" can be delivered into the bloodstream via the nasal route, i.e. by application to the nasal mucosa. Moreover, high bioavailability levels of functional polypeptide or protein in the blood can be achieved after nasal administration at dosage levels which are fully within the limits of tolerability and practicability. Therefore, those polypeptides and proteins can be conveniently administered to a subject by the nasal route by means of a composition comprising said polypeptides or proteins and a pharmaceutically acceptable nasal carrier.

WO2006040153 discloses Nanobodies against amyloid beta and suggests intranasal administration thereof.

WO02074243 discloses expression of scFv antibodies on filamentous phage, and the delivery of the recombinant phage to the central nervous system after intranasal administration.

WO2006091332 discloses the delivery of a mimetibody to the brain after intranasal administration.

### SUMMARY OF THE INVENTION

The subject-matter of the invention is defined in the claims.

Accordingly, described herein is a method for the delivery or administration (both terms are used interchangeably throughout the invention) of a polypeptide or protein or a therapeutic polypeptide or protein to the bloodstream and/or other organ and/or tissue (e.g. the kidney, bladder, lung and/or brain) of a subject without being inactivated (i.e. maintaining functionality), comprising the step of administering to said subject by the nasal route (i.e. nasally or intranasal, both terms are used interchangeably throughout the invention) a composition comprising said polypeptide or protein. The present invention provides a (pharmaceutical) composition (hereafter referred to as the composition or pharmaceutical composition of the invention) comprising a therapeutically effective amount of a (therapeutic) polypeptide or protein and further comprising a pharmaceutically acceptable nasal carrier, for use in a method of treatment comprising systemic delivery of the polypeptide or protein by intranasal administration to the nasal mucosa of a subject. The (therapeutic) polypeptide or protein (hereafter referred to as the polypeptide or protein of the invention or the therapeutic polypeptide or protein of the invention) has an amino acid sequence that consists of one or two Nanobodies, domain antibodies, single domain antibodies or dAbs. In a preferred embodiment, the polypeptide or protein of the invention has an amino acid sequence essentially consisting of a Nanobody, a domain antibody, a single domain antibody or a dAb. In a further preferred embodiment, the polypeptide or protein of the invention has an amino acid sequence essentially consisting of a Nanobody (which is also called the Nanobody of the invention). In a further embodiment, the Nanobody, domain antibody, single domain antibody or dAb is derived from a V_{H} or V_{HH}. As described further in the detailed description, the polypeptide or protein of the invention generally comprises a single amino acid chain that can be considered to comprise "framework sequences" or "FR's" and "complementarity determining regions" or "CDR's".

The application further discloses to use parts, fragments, analogs, mutants, variants, alleles and/or derivatives of the Nanobodies, polypeptides or proteins of the invention, and/or to use polypeptides or proteins comprising or essentially consisting of the same, as long as these are suitable for the uses envisaged herein. Such parts, fragments, analogs, mutants, variants, alleles, derivatives, polypeptides and/or proteins will be described in the further description herein.

According to a specific, but non-limiting embodiment, the amino acid sequences of the Nanobody, polypeptide or protein of the invention can be "humanized", "camelized" or modified as further described herein.

Generally, polypeptides or proteins that comprise or essentially consist of a single Nanobody, domain antibody, single domain antibody or dAb will be referred to herein as "monovalent" polypeptides or proteins or as "monovalent constructs". Polypeptides and proteins that comprise or essentially consist of two or more Nanobodies, domain antibodies, single domain antibodies or dAbs will be referred to herein as "multivalent" polypeptides or proteins, or as "multivalent constructs", and these may provide certain advantages compared to the corresponding monovalent Nanobodies, domain antibodies, single domain antibodies or dAbs.

A polypeptide to be used in the invention may essentially consist of two Nanobodies, domain antibodies, single domain antibodies or dAbs. As further described herein, such multivalent constructs can provide certain advantages compared to a polypeptide or protein comprising or essentially consisting of a single Nanobody, domain antibody, single domain antibody or dAb, such as a much improved affinity and/or specificity for its antigen. Such multivalent constructs will be clear to the skilled person based on the disclosure herein.

A polypeptide to be used in the invention may essentially consist of at least one Nanobody, domain antibody, single domain antibody or dAb directed against one epitope, antigen, target, protein or polypeptide and one other Nanobody, domain antibody, single domain antibody or dAb directed against another epitope, antigen, target, protein or polypeptide. Such polypeptides or proteins are also referred to herein as "multispecific" polypeptides or proteins or as "multispecific constructs", and these may provide certain advantages compared to the corresponding monovalent or monospecific Nanobodies, domain antibodies, single domain antibodies or dAbs. Such multispecific constructs will be clear to the skilled person based on the disclosure herein.

In another aspect of the disclosure, a polypeptide or protein comprises or essentially consists of at least one Nanobody, domain antibody, single domain antibody or dAb, optionally one or more further Nanobodies, domain antibodies, single domain antibodies or dAbs and at least one other amino acid sequence (such as a protein or polypeptide) that confers at least one desired property to the Nanobody, domain antibody, single domain antibody or dAb and/or to the resulting fusion protein. Again, such fusion proteins may provide certain advantages compared to the corresponding monovalent Nanobodies, domain antibodies, single domain antibodies or dAbs. Some non-limiting examples of such amino acid sequences and of such fusion constructs will become clear from the further description herein. In a specific embodiment, said at least one other amino acid sequence provides an increased half-life to the Nanobody, domain antibody, single domain antibody or dAb and/or to the resulting fusion protein. In another specific embodiment, said at least one other amino acid sequence allows the Nanobody, domain antibody, single domain antibody or dAb and/or to the resulting fusion protein to be directed towards, penetrate and/or cross the mucosal membrane and/or the blood brain barrier.

In the above constructs, the one or more Nanobodies, domain antibodies, single domain antibodies or dAbs and/or other amino acid sequences may be directly linked or linked via one or more linker sequences. Some suitable but non-limiting examples of such linkers will become clear from the further description herein.

A polypeptide to be used in the invention comprises one or two Nanobodies, domain antibodies, single domain antibodies or dAbs, optionally linked via one linker, or is a multispecific polypeptide, comprising one Nanobody, domain antibody, single domain antibody or dAbs and at least one Nanobody, domain antibody, single domain antibody or dAb that provides an increased half-life following delivery to the subject, particularly providing extended metabolic persistence in an active state within the physiological environment (e.g., at the nasal mucosal surface, in the bloodstream, and/or within another selected physiological compartment, tissue and/or organ such as e.g. the kidney, bladder, lung and/or brain). Examples are a Nanobody, domain antibody, single domain antibody or dAb directed against a serum protein, and in particular against a human serum protein, such as against human serum albumin, in which said Nanobodies, domain antibodies, single domain antibodies or dAbs again optionally linked via one or more linkers. Such constructs will be clear to the skilled person based on the disclosure herein.

In another embodiment, a polypeptide to be used in the invention comprises one Nanobody, domain antibody, single domain antibody or dAbs linked (optionally via one or more suitable linker sequences) to one amino acid sequence that confers an increased half-life in vivo to the resulting polypeptide of the invention, in particular, that provides extended metabolic persistence in an active state within the physiological environment (e.g. at the nasal mucosal surface, in the bloodstream and/or within another selected physiological compartment, tissue and/or organ such as e.g. the kidney, bladder, lung and/or brain). In particular, said amino acid sequence that confer an increased half-life in vivo to the resulting polypeptide of the invention may be one Nanobody, domain antibody, single domain antibody or dAbs, and in particular Nanobodies, domain antibodies, single domain antibodies or dAbs directed against a human serum protein such as human serum albumin. Examples of suitable Nanobodies against mouse or human serum albumin are described in the applications WO 03/035694, WO 04/041865 and WO 06/122825.

The composition to be used in the present invention is formulated for local administration to the nasal mucosa. Accordingly, in addition to the Nanobody or polypeptide as described above, the composition as used in the invention should also comprise a pharmaceutically acceptable nasal carrier and, optionally, other therapeutic ingredients or pharmaceutically acceptable additives and/or agents.

Thus, in a further aspect, the present invention relates to the use of a composition that comprises at least a Nanobody or polypeptide as described above and a pharmaceutically acceptable nasal carrier. The carrier may be liquid, semi-liquid, a gel, a cream, a paste, a powder or solid, or may be a film or an encapsulation material (microcapsules or nanocapsules). The use of nasal carriers is well known to those skilled in the art of pharmacology. Some non-limiting examples of nasal carriers will become clear from the further description herein.

Optionally, the composition to be used in the invention also comprises other additives and/or agents. Accordingly, in another embodiment of the invention, a composition is provided comprising a Nanobody or polypeptide as described above, a pharmaceutically acceptable nasal carrier and one or more pharmaceutically acceptable additives and/or agents. The use of additives such as preservatives, buffering agents, antioxidants or viscosity builders are known to those skilled in the art of pharmacology and are further described herein. Agents for use in a composition for nasal administration include delivery enhancing agents which are also well known to those skilled in the art of pharmacology and are further described herein.

In various embodiments, the composition to be used in the invention may comprise one or more Nanobodies or polypeptides as described above, one or more pharmaceutically acceptable nasal carriers and, optionally, one or more additives and/or agents.

In another embodiment of the invention, the composition may additionally comprise one or more further therapeutic ingredient (or active substances). In a preferred embodiment, the active substance of the composition to be used in the invention consists of two or more Nanobodies, polypeptides or proteins of the invention. Other embodiments of combined compositions and treatment will become clear from the further description herein.

The present invention also provides methods for the preparation of a composition to be used in the invention. Those methods will also become clear from the further description herein.

The present invention further uses a delivery device, suitable for nasal administration of the composition as described above. Accordingly, the present invention relates to the use of a delivery device comprising the composition as described above and provided with a means enabling the application of said composition to the nasal mucosa. Delivery devices such as provided in the form of drops, a nasal spray, a nasal liquid or powder aerosol, a capsule or a nasal insert are known to the skilled person and will become clear from the further description herein.

Such delivery devices are preferably capable of (repeatedly) delivering a unit dose of the composition as described above, and in particular of (repeatedly) delivering a unit dose of the invention that essentially comprises a delivery volume that is suitable for nasal administration (i.e. as further described herein).

The composition is capable of providing a systemic therapeutic or biological activity of the Nanobody, polypeptide or protein of the invention in a subject, following nasal administration of a composition comprising said Nanobody, polypeptide or protein to said subject. In an embodiment of the invention, the Nanobody or polypeptide to be used in the invention reaches a Cmax in blood of at least 1 ng of Nanobody or polypeptide per ml of blood. In another embodiment, the Nanobody or polypeptide to be used in the invention reaches a Cmax in blood of at least 1 ng of Nanobody or polypeptide per ml of blood following intranasal administration of a dose of 5 mg/kg body weight of said Nanobody or polypeptide. In a further embodiment of the invention, the Nanobody or polypeptide to be used in the invention reaches the bloodstream with a Tmax of less than 120 minutes. In another further embodiment, the Nanobody or polypeptide to be used in the invention reaches a Cmax in blood of at least 1 ng of Nanobody or polypeptide per ml of blood within less than 120 minutes following intranasal administration of the composition comprising said Nanobody or polypeptide. In another further embodiment, the Nanobody or polypeptide to be used in the invention reaches a Cmax in blood of at least 1 ng of Nanobody or polypeptide per ml of blood within less than 120 minutes following intranasal administration of a dose of 5 mg/kg body weight of said Nanobody or polypeptide. In an embodiment of the invention, the AUC for the Nanobody or polypeptide to be used in the invention in blood following intranasal administration of a composition comprising said Nanobody or polypeptide is at least 500 ng/ml/minute Nanobody or polypeptide. In another embodiment of the invention, the AUC for the Nanobody or polypeptide to be used in the invention in blood following intranasal administration of a dose of 5 mg/kg body weight of said Nanobody or polypeptide is at least 500 ng/ml/minute Nanobody or polypeptide. In another further embodiment of the invention, the bioavailability for the Nanobody or polypeptide to be used in the invention in blood following intranasal administration of a composition comprising said Nanobody or polypeptide is at least 1% compared to parenteral administration of said Nanobody or polypeptide.

In an aspect of the disclosure, the composition to be used in the invention is capable of providing a therapeutic or biological activity of the Nanobody or polypeptide to be used in the invention in the brain of a subject, following nasal administration to said subject of a composition comprising said Nanobody or polypeptide. In an aspect of the disclosure, the bioavailability for the Nanobody or polypeptide to be used in the invention in the brain following intranasal administration of a composition comprising said Nanobody or polypeptide is at least 1% compared to parenteral administration of said Nanobody or polypeptide.

The disclosure further provides a method for delivering a polypeptide or protein comprising or essentially consisting of a Nanobody, a domain antibody, a single domain antibody or "dAb" to the bloodstream of a subject without being inactivated, said method comprising the step of nasally administering a Nanobody, polypeptide, protein or composition of the invention to said subject. Also disclosed is a method for delivering a polypeptide or protein comprising or essentially consisting of a Nanobody, a domain antibody, a single domain antibody or "dAb" to the brain of a subject without being inactivated, said method comprising the step of nasally administering a Nanobody, polypeptide, protein or composition of the invention to said subject.

The present disclosure also provides methods for the prevention and/or treatment of a subject in need of a Nanobody, polypeptide or protein of the invention comprising the step of nasally administering to said subject a composition as described above. Further therapeutic applications of the compositions of the invention are described in detail hereafter.

### FIGURES

**Figure 1****:** A, Biodistribution (%ID/g) of ^{99m}Tc tricarbonyl and ^{99m}Tc-FC44 in urine, blood, liver and kidney after 2, 4 and 6 hours following intranasal administration in Wistar rats; B, Biodistribution (%ID/g) of ^{99m}Tc-FC44 in urine, blood, liver and kidney after 2h following intravenous administration in Wistar rats.
**Figure 2****:** SDS-PAGE analysis of urine and kidney extract following intranasal administration of ^{99m}Tc-FC44 in Wistar rats.
**Figure 3****:** Blood levels (%ID/g) of ^{99m}Tc-FC44 following intranasal administration of ^{99m}Tc-FC44 in male Wistar rats.
**Figure 4****:** A, Blood levels (%ID/g) of ^{99m}Tc labeled CEA1, ALB1 and TNF1 Nanobodies 4 hours following intranasal administration in Wistar rats; B, Urine and kidney levels (%ID/g) of ^{99m}Tc labeled CEA1, ALB1 and TNF1 Nanobodies 4 hours following intranasal administration in Wistar rats.
**Figure 5****:** Planar gamma camera activity imaging of a rat immediately after intranasal delivery or oral delivery of ^{99m}Tc-FC44.
**Figure 6****:** Comparison of activity in blood (%ID/g) after intranasal and oral delivery of ^{99m}Tc-FC44.

### DETAILED DESCRIPTION

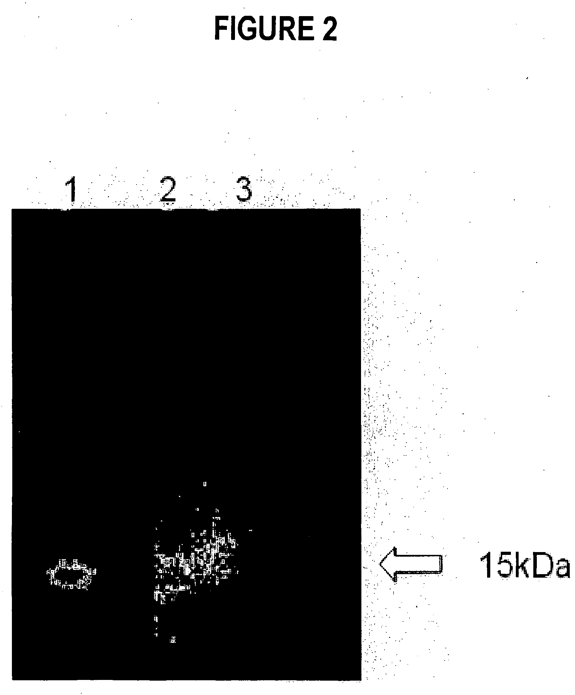

The above and other aspects, embodiments and advantages of the invention will become clear from the further description hereinbelow, in which:
a) Unless indicated or defined otherwise, all terms used have their usual meaning in the art, which will be clear to the skilled person. Reference is for example made to the standard handbooks, such as Sambrook et al, "Molecular Cloning: A Laboratory Manual" (2nd.Ed.), Vols. 1-3, Cold Spring Harbor Laboratory Press (1989); F. Ausubel et al, eds., "Current protocols in molecular biology", Green Publishing and Wiley Interscience, New York (1987); Lewin, "Genes II", John Wiley & Sons, New York, N.Y., (1985); Old et al., "Principles of Gene Manipulation: An Introduction to Genetic Engineering", 2nd edition, University of California Press, Berkeley, CA (1981); Roitt et al., "Immunology" (6th. Ed.), Mosby/Elsevier, Edinburgh (2001); Roitt et al., Roitt's Essential Immunology, 10th Ed. Blackwell Publishing, UK (2001); and Janeway et al., "Immunobiology" (6th Ed.), Garland Science Publishing/Churchill Livingstone, New York (2005), as well as to the general background art cited herein; Rosenfeld G.C., Loose-Mitchell D.S., Pharmacology (3rd Edition) Lippincott Williams & Wilkins, New York; Shlafer M. Pharmacology PreTest™ Self-Assessment and Review (11th Edition) McGraw-Hill Medical Publishing Division, New York; Yang K.Y., Graff L.R., Caughey A.B. Blueprints Pharmacology, Blackwell Publishing.
b) Unless indicated otherwise, all methods, steps, techniques and manipulations that are not specifically described in detail can be performed and have been performed in a manner known per se, as will be clear to the skilled person. Reference is for example again made to the standard handbooks and the general background art mentioned herein and to the further references cited therein;
c) The term "antigenic determinant" refers to the epitope on the antigen recognized by the antigen-binding molecule (such as a Nanobody, a polypeptide or a protein of the invention) and more in particular by the antigen-binding site of said molecule. The terms "antigenic determinant" and "epitope" may also be used interchangeably herein.
d) An amino acid sequence (such as a Nanobody, a polypeptide or a protein of the invention, or generally an antigen binding protein or polypeptide or a fragment thereof) that can bind to, that has affinity for and/or that has specificity for a specific antigenic determinant, epitope, antigen or protein (or for at least one part, fragment or epitope thereof) is said to be "against" or "directed against" said antigenic determinant, epitope, antigen or protein.
e) Unless indicated otherwise, the term "immunoglobulin (sequence)" - whether it is used herein to refer to a heavy chain antibody or to a conventional 4-chain antibody - is used as a general term to include both the full-size antibody, the individual chains thereof, as well as all parts, domains or fragments thereof (including but not limited to antigen-binding domains or fragments such as V_{HH} domains or V_{H}/V_{L} domains, respectively).
f) As further described herein, the amino acid sequence and structure of a Nanobody can be considered - without however being limited thereto - to be comprised of four framework regions or "FR's", which are referred to in the art and herein as "Framework region 1" or "FR1"; as "Framework region 2" or "FR2"; as "Framework region 3" or "FR3"; and as "Framework region 4" or "FR4", respectively; which framework regions are interrupted by three complementary determining regions or "CDR's", which are referred to in the art as "Complementarity Determining Region 1" or "CDR1"; as "Complementarity Determining Region 2" or "CDR2"; and as "Complementarity Determining Region 3" or "CDR3", respectively;
g) As also further described herein, the total number of amino acid residues in a Nanobody can be in the region of 110-120, is preferably 112-115, and is most preferably 113. It should however be noted that parts, fragments, analogs or derivatives (as further described herein) of a Nanobody are not particularly limited as to their length and/or size, as long as such parts, fragments, analogs or derivatives meet the further requirements outlined herein and are also preferably suitable for the purposes described herein;
h) The amino acid residues of a Nanobody are numbered according to the general numbering for V_{H} domains given by Kabat et al. ("Sequence of proteins of immunological interest", US Public Health Services, NIH Bethesda, MD, Publication No. 91), as applied to V_{HH} domains from Camelids in the article of Riechmann and Muyldermans, referred to herein (see for example Figure 2 of said reference).
   According to this numbering, FR1 of a Nanobody comprises the amino acid residues at positions 1-30, CDR1 of a Nanobody comprises the amino acid residues at positions 31-35, FR2 of a Nanobody comprises the amino acids at positions 36-49, CDR2 of a Nanobody comprises the amino acid residues at positions 50-65, FR3 of a Nanobody comprises the amino acid residues at positions 66-94, CDR3 of a Nanobody comprises the amino acid residues at positions 95-102, and FR4 of a Nanobody comprises the amino acid residues at positions 103-113. [In this respect, it should be noted that - as is well known in the art for V_{H} domains and for V_{HH} domains - the total number of amino acid residues in each of the CDR's may vary and may not correspond to the total number of amino acid residues indicated by the Kabat numbering (that is, one or more positions according to the Kabat numbering may not be occupied in the actual sequence, or the actual sequence may contain more amino acid residues than the number allowed for by the Kabat numbering). This means that, generally, the numbering according to Kabat may or may not correspond to the actual numbering of the amino acid residues in the actual sequence. Generally, however, it can be said that, according to the numbering of Kabat and irrespective of the number of amino acid residues in the CDR's, position 1 according to the Kabat numbering corresponds to the start of FR1 and vice versa, position 36 according to the Kabat numbering corresponds to the start of FR2 and vice versa, position 66 according to the Kabat numbering corresponds to the start of FR3 and vice versa, and position 103 according to the Kabat numbering corresponds to the start of FR4 and vice versa].
   Alternative methods for numbering the amino acid residues of V_{H} domains, which methods can also be applied in an analogous manner to V_{HH} domains from Camelids and to Nanobodies, are the method described by Chothia et al. (Nature 342, 877-883 (1989)), the so-called "AbM definition" and the so-called "contact definition"; and
i) The Figures and the Experimental Part/Examples are only given to further illustrate the invention and should not be interpreted or construed as limiting the scope of the invention and/or of the appended claims in any way, unless explicitly indicated otherwise herein.

Without being limited thereto, Nanobodies, (single) domain antibodies or "dAb's" can be derived from the variable region of a 4-chain antibody as well as from the variable region of a heavy chain antibody. In accordance with the terminology used in the references below, the variable domains present in naturally occurring heavy chain antibodies will also be referred to as *"V_{HH} domains",* in order to distinguish them from the heavy chain variable domains that are present in conventional 4-chain antibodies (which will be referred to hereinbelow as *"V_{H} domains"*) and from the light chain variable domains that are present in conventional 4-chain antibodies (which will be referred to hereinbelow as *"V_{L} domains"*).

For a general description of heavy chain antibodies and the variable domains thereof, reference is inter alia made to the following references, which are mentioned as general background art: WO 94/04678, WO 95/04079 and WO 96/34103 of the Vrije Universiteit Brussel; WO 94/25591, WO 99/37681, WO 00/40968, WO 00/43507, WO 00/65057, WO 01/40310, WO 01/44301, EP 1134231 and WO 02/48193 of Unilever; WO 97/49805, WO 01/21817, WO 03/035694, WO 03/054016 and WO 03/055527 of the Vlaams Instituut voor Biotechnologie (VIB); WO 03/050531 of Algonomics N.V. and applicant; WO 01/90190 by the National Research Council of Canada; WO 03/025020 (= EP 1433793) by the Institute of Antibodies; as well as WO 04/041867, WO 04/041862, WO 04/041865, WO 04/041863, WO 04/062551 by applicant and the further published patent applications by applicant; Hamers-Casterman et al., Nature 1993 June 3; 363 (6428): 446-8; Davies and Riechmann, FEBS Lett. 1994 Feb 21; 339(3): 285-90; Muyldermans et al., Protein Eng. 1994 Sep; 7(9): 1129-3; Davies and Riechmann, Biotechnology (NY) 1995 May; 13(5): 475-9; Gharoudi et al., 9th Forum of Applied Biotechnology, Med. Fac. Landbouw Univ. Gent. 1995; 60/4a part I: 2097-2100; Davies and Riechmann, Protein Eng. 1996 Jun; 9(6): 531-7; Desmyter et al., Nat. Struct. Biol. 1996 Sep; 3(9): 803-11; Sheriff et al., Nat. Struct. Biol. 1996 Sep; 3(9): 733-6; Spinelli et al., Nat. Struct. Biol. 1996 Sep; 3(9): 752-7; Arbabi Ghahroudi et al., FEBS Lett. 1997 Sep 15; 414(3): 521-6; Vu et al., Mol. Immunol. 1997 Nov-Dec; 34(16-17): 1121-31; Atarhouch et al., Journal of Camel Practice and Research 1997; 4: 177-182; Nguyen et al., J. Mol. Biol. 1998 Jan 23; 275(3): 413-8; Lauwereys et al., EMBO J. 1998 Jul 1; 17(13): 3512-20; Frenken et al., Res. Immunol. 1998 Jul-Aug; 149(6): 589-99; Transue et al., Proteins 1998 Sep 1; 32(4): 515-22; Muyldermans and Lauwereys, J. Mol. Recognit. 1999 Mar-Apr; 12 (2): 131-40; van der Linden et al., Biochim. Biophys. Acta 1999 Apr 12; 1431(1): 37-46; Decanniere et al., Structure Fold. Des. 1999 Apr 15; 7(4): 361-70; Ngyuen et al., Mol. Immunol. 1999 Jun; 36(8): 515-24; Woolven et al., Immunogenetics 1999 Oct; 50 (1-2): 98-101; Riechmann and Muyldermans, J. Immunol. Methods 1999 Dec 10; 231 (1-2): 25-38; Spinelli et al., Biochemistry 2000 Feb 15; 39(6): 1217-22; Frenken et al., J. Biotechnol. 2000 Feb 28; 78(1): 11-21; Nguyen et al., EMBO J. 2000 Mar 1; 19(5): 921-30; van der Linden et al., J. Immunol. Methods 2000 Jun 23; 240 (1-2): 185-95; Decanniere et al., J. Mol. Biol. 2000 Jun 30; 300 (1): 83-91; van der Linden et al., J. Biotechnol. 2000 Jul 14; 80(3): 261-70; Harmsen et al., Mol. Immunol. 2000 Aug; 37(10): 579-90; Perez et al., Biochemistry 2001 Jan 9; 40(1): 74-83; Conrath et al., J. Biol. Chem. 2001 Mar 9; 276 (10): 7346-50; Muyldermans et al., Trends Biochem. Sci. 2001 Apr; 26(4): 230-5; Muyldermans S., J. Biotechnol. 2001 Jun; 74 (4): 277-302; Desmyter et al., J. Biol. Chem. 2001 Jul 13 ; 276 (28): 26285-90; Spinelli et al., J. Mol. Biol. 2001 Aug 3; 311 (1): 123-9; Conrath et al., Antimicrob. Agents Chemother. 2001 Oct; 45 (10): 2807-12; Decanniere et al., J. Mol. Biol. 2001 Oct 26; 313(3): 473-8; Nguyen et al., Adv. Immunol. 2001; 79: 261-96; Muruganandam et al., FASEB J. 2002 Feb; 16 (2): 240-2; Ewert et al., Biochemistry 2002 Mar 19; 41 (11): 3628-36; Dumoulin et al., Protein Sci. 2002 Mar; 11 (3): 500-15; Cortez-Retamozo et al., Int. J. Cancer 2002 Mar 20; 98 (3): 456-62; Su et al., Mol. Biol. Evol. 2002 Mar; 19 (3): 205-15; van der Vaart JM., Methods Mol. Biol. 2002; 178: 359-66; Vranken et al., Biochemistry 2002 Jul 9; 41 (27): 8570-9; Nguyen et al., Immunogenetics 2002 Apr; 54 (1): 39-47; Renisio et al., Proteins 2002 Jun 1; 47 (4): 546-55; Desmyter et al., J. Biol. Chem. 2002 Jun 28; 277 (26): 23645-50; Ledeboer et al., J. Dairy Sci. 2002 Jun; 85 (6): 1376-82; De Genst et al., J. Biol. Chem. 2002 Aug 16; 277 (33): 29897-907; Ferrat et al., Biochem. J. 2002 Sep 1; 366 (Pt 2): 415-22; Thomassen et al., Enzyme and Microbial Technol. 2002; 30: 273-8; Harmsen et al., Appl. Microbiol. Biotechnol. 2002 Dec; 60 (4): 449-54; Jobling et al., Nat. Biotechnol. 2003 Jan; 21 (1): 77-80; Conrath et al., Dev. Comp. Immunol. 2003 Feb; 27 (2): 87-103; Pleschberger et al., Bioconjug. Chem. 2003 Mar-Apr; 14 (2): 440-8; Lah et al., J. Biol. Chem. 2003 Apr 18; 278 (16): 14101-11; Nguyen et al., Immunology 2003 May; 109 (1): 93-101; Joosten et al., Microb. Cell Fact. 2003 Jan 30; 2 (1): 1; Li et al., Proteins 2003 Jul 1; 52 (1): 47-50; Loris et al., Biol. Chem. 2003 Jul 25; 278 (30): 28252-7; van Koningsbruggen et al., J. Immunol. Methods 2003 Aug; 279 (1-2): 149-61; Dumoulin et al., Nature 2003 Aug 14; 424 (6950): 783-8; Bond et al., J. Mol. Biol. 2003 Sep 19; 332 (3): 643-55; Yau et al., J. Immunol. Methods 2003 Oct 1; 281 (1-2): 161-75; Dekker et al., J. Virol. 2003 Nov; 77 (22): 12132-9; Meddeb-Mouelhi et al., Toxicon 2003 Dec; 42 (7): 785-91; Verheesen et al., Biochim. Biophys. Acta 2003 Dec 5; 1624 (1-3): 21-8; Zhang et al., J. Mol. Biol. 2004 Jan 2; 335 (1): 49-56; Stijlemans et al., J. Bio.l Chem. 2004 Jan 9; 279 (2): 1256-61; Cortez-Retamozo et al., Cancer Res. 2004 Apr 15; 64 (8): 2853-7; Spinelli et al., FEBS Lett. 2004 Apr 23; 564 (1-2): 35-40; Pleschberger et al., Bioconjug. Chem. 2004 May-Jun; 15 (3): 664-71; Nicaise et al., Protein Sci. 2004 Jul; 13 (7): 1882-91; Omidfar et al., Tumour Biol. 2004 Jul-Aug; 25 (4): 179-87; Omidfar et al., Tumour Biol. 2004 Sep-Dec; 25(5-6): 296-305; Szynol et al., Antimicrob. Agents Chemother. 2004 Sep;48(9):3390-5; Saerens et al., J. Biol. Chem. 2004 Dec 10; 279 (50): 51965-72; De Genst et al., J. Biol. Chem. 2004 Dec 17; 279 (51): 53593-601; Dolk et al., Appl. Environ. Microbiol. 2005 Jan; 71(1): 442-50; Joosten et al., Appl. Microbiol. Biotechnol. 2005 Jan; 66(4): 384-92; Dumoulin et al., J. Mol. Biol. 2005 Feb 25; 346 (3): 773-88; Yau et al., J. Immunol. Methods. 2005 Feb; 297 (1-2): 213-24; De Genst et al., J. Biol. Chem. 2005 Apr 8; 280 (14): 14114-21; Huang et al., Eur. J. Hum. Genet. 2005 Apr 13; Dolk et al., Proteins 2005 May 15; 59 (3): 555-64; Bond et al., J. Mol. Biol. 2005 May 6; 348(3): 699-709; Zarebski et al., J. Mol. Biol. 2005 Apr 21; [E-publication ahead of print].

For a general description of (single) domain antibodies or "dAb's", reference is made to Ward et al. (Nature 1989 Oct 12; 341 (6242): 544-6), EP 0 368 684 and Holliger and Hudson (Nature Biot. 2005, 23: 1126-1136). In particular, the amino acid sequence of (single) domain antibodies or "dAb's" can be considered - without however being limited thereto - to be comprised of four framework regions or "FR's" and three complementary determining regions of "CDR's" as described above for Nanobodies.

Thus - without being limited thereto - the polypeptide to be used in the invention has an amino acid sequence that comprises or essentially consists of four framework regions (FR1 to FR4, respectively) and three complementarity determining regions (CDR1 to CDR3, respectively). Such an amino acid sequence preferably contains between 80 and 200 amino acid residues, such as between 90 and 150 amino acid residues, such as about 100-130 amino acid residues (although suitable fragments of such an amino acid sequence - i.e. essentially as described herein for the Nanobodies - may also be used), and is preferably such that it forms an immunoglobulin fold or such that, under suitable conditions, it is capable of forming an immunoglobulin fold (i.e. by suitable folding). The amino acid sequence is preferably chosen from Nanobodies, domain antibodies, single domain antibodies or "dAb's", and is most preferably a Nanobody as defined herein. The CDR's may be any suitable CDR's that provide the desired property to the polypeptide or protein.

A further description of the Nanobodies, polypeptides and proteins in their broadest sense, including humanization and/or camelization of Nanobodies, as well as other modifications, parts or fragments, derivatives or "Nanobody fusions", multivalent constructs (including some non-limiting examples of linker sequences) and different modifications to increase the half-life of the Nanobodies, polypeptides and proteins can be found e.g. in WO 06/040153, WO 06/122825, WO 06/122786, WO 07/042289, WO 07/104529 and WO2008020079. A detailed description of the preparation of the Nanobodies, polypeptides and proteins of the invention can be found e.g. in WO 06/040153, WO 06/122825, WO 06/122786, WO 07/042289, WO 07/104529 and WO2008020079 as well.

In an aspect of the disclosure, the Nanobodies, polypeptides or proteins may also form a sequence or signal that allows the Nanobody, polypeptide or protein to be directed towards and/or to penetrate or enter into specific mucosal cells, or parts or compartments of said cells, and/or that allows the Nanobody, polypeptide or protein of the invention to penetrate or cross a biological barrier such as a mucosal membrane, a cell layer of the mucosal membrane.

In another aspect of the disclosure, the polypeptide or protein is a multispecific polypeptide comprising at least one Nanobody, domain antibody, single domain antibody or dAb directed against a target and at least one Nanobody, domain antibody, single domain antibody or dAb that directs the polypeptide or protein towards, and/or that allows the polypeptide or protein to penetrate or to enter into specific mucosal cells, or parts or compartments of said cells, and/or that allows the polypeptide or protein to penetrate or cross a biological barrier such as the mucosal membrane or a cell layer of said mucosal membrane. Examples of such Nanobodies, domain antibodies, single domain antibodies or dAbs include Nanobodies, domain antibodies, single domain antibodies or dAbs that are directed towards specific cell-surface proteins, markers or epitopes of the mucosal membrane cells.

In this context, the polypeptide or protein may comprise one or more Nanobodies, domain antibodies, single domain antibodies or dAbs directed against the desired target and one or more ligand (also called membrane crossing ligand) directed against an epithelial transmembrane protein on the mucosal membrane, wherein said polypeptide or protein crosses the mucosal membrane upon binding of the ligand to said epithelial transmembrane protein.

An epithelial transmembrane protein is a protein or receptor displayed on the mucosal membrane which upon binding to a ligand, mediates the transport of said ligand through the mucosal membrane.

In one aspect of the disclosure, the ligand is a Nanobody, domain antibody, single domain antibody or dAb directed against an epithelial transmembrane protein on the mucosal membrane. The polypeptide or protein crosses the mucosal membrane upon binding of said Nanobody, domain antibody, single domain antibody or dAb to said epithelial transmembrane protein. The membrane crossing Nanobody, domain antibody, single domain antibody or dAb may be prepared from a peptide library which is screened for binding to the epithelial transmembrane protein or for crossing properties.

In another aspect of the disclosure, the polypeptide construct comprises a therapeutic polypeptide or agent which is covalently or non-covalently linked to a Nanobody, domain antibody, single domain antibody or dAb directed against an epithelial transmembrane protein on the mucosal membrane. It is an aspect of the disclosure that these Nanobodies, domain antibodies, single domain antibodies or dAbs can be added as a tag to polypeptides or proteins of the invention, for crossing or passage through the mucosal membrane.

In yet another aspect of the disclosure, the Nanobody, polypeptide or protein directed against the desired target is linked to another ligand (e.g. a natural ligand) of the epithelial transmembrane protein. The resulting construct, upon binding of the ligand to the epithelial transmembrane protein, is transported through the mucosal membrane.

Another aspect of the disclosure is a method for selecting Nanobodies, domain antibodies, single domain antibodies or dAbs directed against an epithelial transmembrane protein, wherein said Nanobody, domain antibody, single domain antibody or dAb crosses the mucosal membrane upon binding to said epithelial transmembrane protein. Said method comprises panning epithelial transmembrane protein-displaying membranes with a phage library (naive or immune) of Nanobodies, domain antibodies, single domain antibodies or dAbs, and selecting for membrane crossing Nanobodies, domain antibodies, single domain antibodies or dAbs by recovering the transported phage from the membrane. The disclosure includes a selection method which uses cell lines that overexpress an epithelial transmembrane protein or cell lines transfected with an epithelial transmembrane protein gene to allow the easy selection of phage Nanobodies, domain antibodies, single domain antibodies or dAbs binding to the epithelial transmembrane protein. This avoids the need for protein expression and purification, speeding up significantly the generation of membrane crossing Nanobodies, domain antibodies, single domain antibodies or dAbs. In another embodiment, the disclosure includes a selection method using cells to allow the selection of phage Nanobodies, domain antibodies, single domain antibodies or dAbs that show receptor mediated internalization. Said method comprises adding the phage Nanobodies, domain antibodies, single domain antibodies or dAbs to the cells and recovering the phage Nanobodies, domain antibodies, single domain antibodies or dAbs from the cells that have undergone internalization. In yet another embodiment, the disclosure includes a selection method using cells seeded on a filter or in a Transwell system or Boyden chamber to allow the selection of phage Nanobodies, domain antibodies, single domain antibodies or dAbs that transcytose through the cell monolayer. Said method comprises adding the phage Nanobodies, domain antibodies, single domain antibodies or dAbs to compartment 1, allow the phage Nanobodies, domain antibodies, single domain antibodies or dAbs to migrate across the cell monolayer and harvest the phage Nanobodies that migrate in compartment 2.

Generally, the Nanobody, polypeptide or protein can be formulated in any suitable manner known per se, that allows said Nanobody, polypeptide or protein to be nasally administered and for which reference is made to standard handbooks, such as Remington's Pharmaceutical Sciences, 18th Ed., Mack Publishing Company, USA (1990) or Remington, the Science and Practice of Pharmacy, 21th Edition, Lippincott Williams and Wilkins (2005). Preferably, for nasal administration, the Nanobodies, polypeptides and proteins are formulated as a composition or pharmaceutical composition comprising at least a therapeutically effective amount of the Nanobody, polypeptide or protein and at least one pharmaceutically acceptable nasal carrier, and optionally one or more further therapeutic ingredients (or active substances), pharmaceutically acceptable additives and/or agents. A "nasal carrier" as defined in the present invention is a carrier that is suitable for application through the nasal route, i.e. application to the nasal mucosa. The nasal carrier may be a solid, semi-liquid or liquid filler, diluent or encapsulating material. The nasal carrier can be provided in a variety of forms, including, fluid or semi-liquid or viscous solutions, gels, creams, pastes, powders, microspheres and films for direct application to the nasal mucosa. The nasal carrier should be "pharmaceutically acceptable" in the sense of being compatible with the other ingredients of the formulation and not eliciting an unacceptable deleterious effect in the subject. Preferred nasal carriers are those which improve the contact of the Nanobody, polypeptide or protein with the nasal mucosa or facilitate the diffusion of the Nanobody, polypeptide or protein from the composition to the nasal mucosa, e.g. which prolong the nasal residence time of the composition and/or reduce the distance between the Nanobody, polypeptide or protein and the mucosa, allowing increased absorption. Suitable nasal carriers are described hereafter or are otherwise well known to those skilled in the art of pharmacology. Such carriers may be used in suitable amounts known per se, which will be clear to the skilled person based on the disclosure and prior art cited herein.

The carrier can be a liquid or solvent or dispersion medium containing, for example, water, ethanol, polyol (for example, glycerol, propylene glycol, and liquid polyethylene glycol, and the like), and suitable mixtures thereof. Other preferred liquid carriers are aqueous saline, e.g. physiological saline, or an aqueous buffer, e.g. a phosphate/citric acid buffer. Non-limiting examples of other liquid nasal carriers are provided in WO 04/093917 and WO 05/120551.

Where a solid carrier is present, it may be e.g. water-insoluble, sparingly water soluble, water absorbing, water swellable, gel forming or water soluble. For solid compositions, conventional nontoxic pharmaceutically acceptable carriers can be used which include, pharmaceutical grades of mannitol, lactose, starch, magnesium stearate, sodium saccharin, talcum, cellulose, glucose, sucrose, magnesium carbonate, and the like. Other preferred carriers include polyacrylates, sodium carboxy methyl cellulose, starches and their derivatives, alginic acid and salts, hyaluronic acid and salts, pectic acid and salts, gelatin and its derivatives, gums, polylactic acid and its copolymers, polyvinyl acetate, the celluloses and their derivatives, coated celluloses, crosslinked dextrans, more preferably polylactic acid and its copolymers, polyvinyl acetate, celluloses and their derivatives, coated celluloses and crosslinked dextrans. Non-limiting examples of other solid nasal carriers are described in EP 490806, WO 04/093917 and WO 05/120551.

Release of the Nanobody, polypeptide or protein may be by diffusion or disintegration of the nasal carrier. In some circumstances, the Nanobody, polypeptide or protein is dispersed in microcapsules (microspheres) or nanocapsules (nanospheres) prepared from a suitable polymer, e.g., isobutyl 2-cyanoacrylate (see, e.g., Michael et al., J. Pharmacy Pharmacol. 1991; 43: 1-5). These particular microspheres not only demonstrate mucoadhesion properties, but also protect against enzymatic degradation. They further allow manipulation of Nanobody, polypeptide or protein release rates yielding sustained delivery and biological activity over a protracted time (Morimoto et al., Eur. J. Pharm. Sci. 2001 May; 13(2): 179-85).

Another technique aimed to increase nasal absorption is the utilization of bioadhesive nasal carriers. These compounds adhere to the nasal mucosa by chemical or physical binding such as Van der Waals interaction, ionic interaction, hydrogen bonding or by polymer chain entanglement. The adhesion may take place to the epithelial (cellular) surface or to the mucus overlying the surface. These compounds promote binding of drugs to biological material in the nasal cavity, thereby extending residence time and allowing increased absorption. Common bioadhesive materials are carbopol, cellulose agents, starch, dextran, and chitosan (Illum, Bioadhesive formulations for nasal peptide delivery. *In*: E Mathiowitz, DE Chickering III, C Lehr, eds. Bioadhesive Drug Delivery Systems. New York: Marcel Dekker, 1999: 507-541; WO 96/03142).

To further enhance mucosal delivery of the Nanobody, polypeptide or protein of the invention, formulations comprising said Nanobody, polypeptide or protein may also contain a hydrophilic low molecular weight compound as a nasal carrier, base or excipient. Such hydrophilic low molecular weight compounds provide a passage medium through which a water-soluble active agent, such as the Nanobody, polypeptide or protein , may diffuse through the base to the nasal mucosa where said active Nanobody, polypeptide or protein is absorbed. The hydrophilic low molecular weight compound optionally absorbs moisture from the mucosa or the administration atmosphere and dissolves the water-soluble active Nanobody, polypeptide or protein. Exemplary hydrophilic low molecular weight compound are disclosed in WO 05/120551 and include polyol compounds, such as oligo-, di- and monosaccarides such as sucrose, mannitol, lactose, L-arabinose, D-erytrose, D-ribose, D-xylose, D-mannose, D-galactose, lactulose, cellobiose, gentibiose, glycerin and polyethylene glycol. Other non-limiting examples of hydrophilic low molecular weight compounds useful as such carriers or bases include N-methylpyrrolidone, and alcohols (e.g. oligovinyl alcohol, ethanol, ethylene glycol, propylene glycol, etc.). These hydrophilic low molecular weight compounds can be used alone or in combination with one another or with other active or inactive components of the intranasal formulation.

A carrier may further contain pharmaceutically acceptable additives such as acidifying agents, alkalizing agents, antimicrobial preservatives, antioxidants, buffering agents, chelating agents, complexing agents, solubilizing agents, humectants, solvents, suspending and/or viscosity- increasing agents, stabilizers, tonicity adjustors, wetting agents or other biocompatible materials. A tabulation of ingredients listed by the above categories, can be found in the U. S. Pharmacopeia National Formulary, pp. 1857-1859, 1990. Examples of pharmaceutically acceptable preservatives include benzalkonium chloride, an alkyl p-hydroxybenzoate (paraben) such as methyl p-hydroxybenzoate and propyl p-hydroxybenzoate, or sodium methylmercurithiosalicylate (Thiomersal). Further non-limiting examples of pharmaceutically acceptable preservatives are described in US 5,759,565, EP 490806 and WO 04/093917. Examples of pharmaceutically acceptable antioxidants include alkali metal sulfites, alkali metal bisulfites, alkali metal pyrosulfites, sodium thiosulfate, thiodipropionic acid, cysteine in free or salt form, such as cysteine hydrochloride, ascorbic acid, citraconic acid, propyl or ethyl gallate, nordihydroguaiaretic acid, butylated hydroxyanisole or -toluene, tocol. Further non-limiting examples of pharmaceutically acceptable antioxidants are provided in EP 490806, WO 04/093917 and WO 05/120551. Examples of pharmaceutically acceptable viscosity builders include methylcellulose, hydroxymethylcellulose, PVA, PVP, polyacrylic acid or natural polymers. Further non-limiting examples of viscosity builders are described in EP 490806. Examples of pharmaceutically acceptable stabilizers include albumin, e.g. human serum albumin, aprotinin or ε-aminocaproic acid. Further non-limiting examples of stabilizers are provided in EP 490806. Examples of pharmaceutically acceptable tonicity adjustors include nasally acceptable sugars, e.g. glucose, mannitol, sorbitol, ribose, mannose, arabinose, xylose or another aldose or glucosamine. Further non-limiting examples of tonicity adjustors are provided in EP 490806. Such additives may be used in suitable amounts known per se, which will be clear to the skilled person based on the disclosure and prior art cited herein.

When the nasal carrier is a liquid, the tonicity of the formulation, as measured with reference to the tonicity of 0.9% (w/v) physiological saline solution taken as unity, is typically adjusted to a value at which no substantial, irreversible tissue damage will be induced in the nasal mucosa at the site of administration. Generally, the tonicity of the solution is adjusted to a value of about 1/3 to 3, more typically 1/2 to 2, and most often 3/4 to 1.7. Liquid compositions of the invention further preferably have a mildly acid pH, e.g. from 3 to 6.5, from 3.5 to 6.5, preferably from 4.5 to 6.5 to minimize nasal irritation (Behl et al. Adv. Drug Delivery Rev. 1998; 29: 89-116). The required degree of acidity may conveniently be achieved, e.g by the addition of a buffering agent, e.g. a mixture of citric acid and disodium hydrogen phosphate, or an acid such as HCl or another appropriate mineral or an organic acid, e.g. phosphoric acid. Solid compositions may also comprise a buffering agent when they are prepared by lyophilization of a liquid composition buffered to a pH value as indicated above. Non-limiting examples of pharmaceutically acceptable buffering agents are provided in EP 490806, WO 04/093917 and WO 05/120551.

The desired viscosity for the compositions to be used in the invention will depend on the particular form for administration, e.g. whether administration is to be by nasal drops or nasal spray. For nasal drops an appropriate viscosity is from about 2 to about 40×10⁻³ Pa s. For nasal sprays the viscosity will suitably be less than 2×10⁻³ Pa s, e.g. from 1 to 2×10⁻³ Pa s.

The particle size of the components including the carriers, e.g. the cellulose carrier, in the solid nasal composition of the invention may be from 5 to 500 µ, preferably from 10 to 250 µ, more preferably from 20 to 200 µ. For example, the average particle size may be in the range of 50 to 100 µ.

Alternatively, the Nanobodies, polypeptides or proteins may also be suitably formulated per se for intranasal delivery, for example in the form of a powder (such as a freeze-dried or micronised powder) or mist; for example with a particle size within the ranges indicated herein.

The composition to be used in the invention may further comprise one or more mucosal delivery enhancing agents. As used herein, "mucosal delivery-enhancing agents" include agents which enhance the release or solubility (e.g., from a formulation delivery vehicle), diffusion rate, penetration capacity and timing, uptake, residence time, stability, effective half-life, peak or sustained concentration levels, clearance and other desired mucosal delivery characteristics (e.g., as measured at the site of delivery, or at a selected target site of activity such as the bloodstream and/or another selected physiological compartment, tissue and/or organ such as e.g. the kidney, bladder, lung and/or brain) of the Nanobody, polypeptide or protein of the invention or of additional biologically active ingredient(s). Enhancement of mucosal delivery can thus occur by any of a variety of mechanisms, for example by increasing the diffusion, transport, persistence or stability of the Nanobody, polypeptide or protein, enzyme inhibition, increasing membrane fluidity, modulating the availability or action of calcium and other ions that regulate intracellular or paracellular permeation, solubilizing mucosal membrane components (e.g., lipids), changing non-protein and protein sulfhydryl levels in mucosal tissues, increasing water flux across the mucosal surface, modulating epithelial junctional physiology, reducing the viscosity of mucus overlying the mucosal epithelium, reducing mucociliary clearance rates, increasing nasal blood flow and other mechanisms. Suitable mucosal delivery enhancing agents will be clear to a person skilled in the art of pharmacology and are further described hereafter. Such mucosal delivery agents may be used in suitable amounts known per se, which will be clear to the skilled person based on the disclosure and prior art cited herein.

Mucosal delivery enhancing agents include (a) aggregation inhibitory agents, (b) charge modifying agents, (c) pH control agents, (d) degradative enzyme inhibitors; (e) mucolytic or mucus clearing agents, (f) ciliostatic agents; (g) membrane penetration-enhancing agents, (h) modulatory agents of epithelial junction physiology, such as nitric oxide (NO) stimulators, chitosan, and chitosan derivatives; (i) vasodilator agents, (j) selective transport-enhancing agents, and (k) stabilizing delivery vehicles, carriers, supports or complex-forming species with which the Nanobody, polypeptide or protein of the invention is effectively combined, associated, contained, encapsulated or bound to stabilize the Nanobody, polypeptide or protein of the invention for enhanced nasal mucosal delivery. These mucosal delivery enhancing agents are further exemplified - without being limiting as additional agents comprised in the compositions of the present invention - in EP 037943, EP 094157, EP 173990, EP 214898, EP 215697, EP 327756, EP 490806, US 4,476,116, US 5,759,565, WO 04/093917 and WO 05/120551.

In one embodiment, anti-aggregation agents are added to the composition to be used in the invention. Aggregation inhibitory agents include, for example, polymers of various functionalities, such as polyethylene glycol, dextran, diethylaminoethyl dextran, and carboxymethyl cellulose, which significantly increase the stability and reduce the solid-phase aggregation of peptides and proteins admixed therewith or linked thereto. In some instances, the activity or physical stability of proteins can also be enhanced by various additives to aqueous solutions of the Nanobody, peptide or protein drugs. For example, additives, such as polyols (including sugars), amino acids, and various salts may be used. Certain additives, in particular sugars and other polyols, also impart significant physical stability to dry, e.g., lyophilized proteins. These additives can also be used within the invention to protect the proteins against aggregation not only during lyophilization but also during storage in the dry state. For example, sucrose and Ficoll 70 (a polymer with sucrose units) exhibit significant protection against peptide or protein aggregation during solid-phase incubation under various conditions. These additives may also enhance the stability of solid proteins embedded within polymer matrices. Yet additional additives, for example sucrose, stabilize proteins against solid-state aggregation in humid atmospheres at elevated temperatures, as may occur in certain sustained-release formulations of the invention. These additives can be incorporated into polymeric melt processes and compositions within the invention. For example, polypeptide microparticles can be prepared by simply lyophilizing or spray drying a solution containing various stabilizing additives described above. Sustained release of unaggregated peptides and proteins can thereby be obtained over an extended period of time. A wide non-limiting range of suitable methods and anti-aggregation agents are available for incorporation within the compositions to be used in the invention such as disclosed in WO 05/120551, Breslow et al. (J. Am. Chem. Soc. 1996;118: 11678-11681), Breslow et al.(PNAS USA 1997; 94: 11156-11158), Breslow et al. (Tetrahedron Lett. 1998; 2887-2890), Zutshi et al. (Curr. Opin. Chem. Biol. 1998; 2: 62-66), Daugherty et al. (J. Am. Chem. Soc. 1999; 121: 4325-4333), Zutshi et al. (J. Am. Chem. Soc. 1997; 119: 4841-4845), Ghosh et al. (Chem. Biol. 1997; 5: 439-445), Hamuro et al. (Angew. Chem. Int. Ed. Engl. 1997; 36: 2680-2683), Alberg et al., Science 1993; 262: 248-250), Tauton et al. (J. Am. Chem. Soc. 1996; 118: 10412-10422), Park et al. (J. Am. Chem. Soc. 1999; 121: 8-13), Prasanna et al. (Biochemistrv 1998; 37:6883-6893), Tiley et al. (J. Am. Chem. Soc. 1997; 119: 7589-7590), Judice et al. (PNAS USA 1997; 94: 13426-13430), Fan et al. (J. Am. Chem. Soc. 1998; 120: 8893-8894), Gamboni et al. (Biochemistry 1998; 37: 12189-12194).

In another embodiment, enzyme inhibitors are added to the composition to be used in the invention. The nasal mucosa contains hydrolytic enzymes, such as lipases and proteases, which must be overcome. This enzymatic "barrier" can be dampened by administering enzyme inhibitors that prevent or at least lessen the extent of degradation. Enzyme inhibitors for use within the invention are selected from a wide range of non-protein inhibitors that vary in their degree of potency and toxicity (see, e.g., L. Stryer, Biochemistry, WH: Freeman and Company, NY, NY, 1988). Non-limiting examples include amastatin and bestatin (O'Hagan et al., Pharm. Res. 1990, 7: 772-776). Various classes of enzyme inhibitors are extensively described and exemplified in WO 05/120551 without being limiting for use in the composition to be used in the present invention. Another means to inhibit degradation is pegylation with PEG molecules, preferably low molecular weight PEG molecules (e.g. 2 kDa; Lee et al., Calcif Tissue Int. 2003, 73: 545-549). Also described is the use, as enzyme inhibitor, of a Nanobody, domain antibody, single domain antibody or "dAb" directed against said enzyme. Accordingly, the invention also relates to a bispecific or multispecific construct essentially consisting of one or two Nanobodies, domain antibodies, single domain antibodies or "dAbs" directed against the desired target and one or more Nanobodies, domain antibodies, single domain antibodies or "dAbs" directed against an enzyme of the nasal mucosa.

In another embodiment, a mucolytic agent is added to the composition to be used in the invention to further decrease the viscosity of the nasal mucosa. Mucolytic agents are described in WO 04/093917 and include n-acetyl-cysteine, propyl gallate and cysteine methionine dimers that compete by mass action for sulfhydryls and disulfide bonds in the mucous polymer. As disulfide bonds are broken, the overall viscosity of the mucus is reduced significantly. Other forms of secondary structure in the mucosa are cooperative hydrogen bonded structures involving polysaccharide and aminosaccharide chains. This cooperative hydrogen bonding is reduced with the chaotropes, and it is actually the free energy of the increased entropy from the hydrogen bonded ordered structures as they become randomized that is contributed as a pleasant sensation of warmth in the nostril during application of the compositions comprising these compounds.

In a further embodiment, a membrane penetration-enhancing agent is added to the composition to be used in the present invention. Different membrane penetration-enhancing agents have been described such as (i) a surfactant, (ii) a bile salt or bile salt derivative, (iii) a phospholipid or fatty acid additive, mixed micelle, liposome, or carrier, (iv) an alcohol, (v) an enamine, (vi) an NO donor compound, (vii) a long-chain amphipathic molecule (viii) a small hydrophobic penetration enhancer, (ix) sodium or a salicylic acid derivative, (x) a glycerol ester of acetoacetic acid, (xi) a cyclodextrin or beta-cyclodextrin derivative, (xii) a medium-chain fatty acid, (xiii) a chelating agent (e.g., citric acid, salicylates), (xiv) an amino acid or salt thereof, (xv) an N-acetylamino acid or salt thereof, (xvi) an enzyme degradative to a selected membrane component, (xvii) an inhibitor of fatty acid synthesis, (xviii) an inhibitor of cholesterol synthesis, (xix) cationic polymers, or (xx) any combination of the membrane penetration enhancing agents of ((i)-(xix)). The membrane penetration-enhancing agent can be selected from small hydrophilic molecules, including but not limited to, dimethyl sulfoxide (DMSO), dimethylformamide, ethanol, propylene glycol, and the 2-pyrrolidones. Alternatively, long-chain amphipathic molecules, for example, deacylmethyl sulfoxide, azone, sodium lauryl sulfate, oleic acid, and the bile salts (e.g., unsaturated cyclic ureas and Transcutol), may be employed to enhance mucosal penetration of the Nanobodies, polypeptides or proteins of the invention. In additional aspects, surfactants (e.g., Tween 80, Poloxamer 188, polysorbates; further non-limiting examples of surfactants are also provided in EP 490806, US 5,759,565, and WO 04/093917) are employed as adjunct compounds, processing agents, or formulation additives to enhance intranasal delivery of the Nanobodies or polypeptides described above. These penetration-enhancing agents typically interact at either the polar head groups or the hydrophilic tail regions of molecules that comprise the lipid bilayer of epithelial cells lining the nasal mucosa (Barry, Pharmacology of the Skin, Vol. 1, pp. 121-137, Shroot et al., Eds., Karger, Basel, 1987; and Barry, J. Controlled Release 1987; 6: 85-97). Interaction at these sites may have the effect of disrupting the packing of the lipid molecules, increasing the fluidity of the bilayer, and facilitating transport of the Nanobodies or polypeptides described above across the mucosal barrier. Additional non-limiting examples of membrane penetration-enhancing agent are described in WO 04/093917, WO 05/120551 and Davis and Ilium (Clin. Pharmacokinet 2003, 42: 1107-1128).

In various embodiments of the invention, the Nanobody or polypeptide to be used in the invention is combined with one, two, three, four or more of the mucosal (e.g., intranasal) delivery- enhancing agents recited in (a)-(k) above. These mucosal delivery-enhancing agents may be admixed, alone or together, with the nasal carrier and with the Nanobody, polypeptide or protein, or otherwise combined therewith in a pharmaceutically acceptable formulation or delivery vehicle. Formulation of the Nanobody or polypeptide with one or more of the mucosal delivery-enhancing agents according to the teachings herein (optionally including any combination of two or more mucosal delivery-enhancing agents selected from (a)-(k) above) provides for increased bioavailability of the Nanobody or polypeptide following delivery thereof to the nasal mucosal surface of a subject.

While the mechanism of absorption promotion may vary with different intranasal delivery-enhancing agents of the invention, useful reagents in this context will not substantially adversely affect the mucosal tissue and will be selected according to the physicochemical characteristics of the particular Nanobody, polypeptide or protein or other active ingredients or delivery enhancing agent. In this context, delivery-enhancing agents that increase penetration or permeability of mucosal tissues will often result in some alteration of the protective permeability barrier of the mucosa. For such delivery-enhancing agents to be of value within the invention, it is generally desired that any significant changes in permeability of the mucosa be reversible within a time frame appropriate to the desired duration of drug delivery. Furthermore, there should be no substantial, cumulative toxicity, nor any permanent deleterious changes induced in the barrier properties of the mucosa with long term use.

In addition to the Nanobody or polypeptide to be used in the invention, the nasal carrier and, optionally, one or more further additives and/or agents, the composition to be used in the invention may further comprise one or more additional therapeutic ingredients (or active substances). These therapeutic ingredients can be any compound that elicits a desired activity or therapeutic or biological response in the subject. In a preferred embodiment, two or more Nanobodiesmay be used in combination, i.e. as a combined treatment regimen.

As indicated above, the composition or pharmaceutical composition to be used in the invention should comprise at least a therapeutically effective amount of the Nanobody or polypeptide described above. A "therapeutically effective amount" as used in the present invention in its broadest sense means an amount of the Nanobody or polypeptide that is capable of eliciting the desired activity or the desired biological, prophylactic and/or therapeutic response in the subject receiving said Nanobody or polypeptide. The amount of Nanobody or polypeptide to be administered and hence the amount of active ingredient in the composition to be used in the invention will, of course, vary according to factors such as the bioavailability of the compound, the disease indication and particular status of the subject (e.g., the subject's age, size, fitness, extent of symptoms, susceptibility factors, etc), the target cell, tumor, tissue, graft or organ, other drugs or treatments being administered concurrently, as well as the specific pharmacology of the Nanobodies or polypeptides for eliciting the desired activity or biological, prophylatctic or therapeutic response in the subject. Dosage regimens may be adjusted to provide an optimum activity or biological, prophylactic or therapeutic response. Dosages should also be adjusted based on the release rate of the administered formulation (e.g. a nasal spray versus powder). A therapeutically effective amount is also one in which any toxic or detrimental side effects of the Nanobody or polypeptide is outweighed in clinical terms by therapeutically beneficial effects. Doses may be chosen to be equipotent to the injection route.

In this context, the absolute bioavailability of the Nanobody or polypeptide to be used in the invention following nasal administration of the composition is of the order of ca. 1, 2, 3, 5, 7, 10, 15, 20, 25, 30, 40, 50, 60, 70, 80, 90, 100% or more of the levels achieved on injection. Absolute bioavailability measures the availability of the active drug in systemic circulation after intranasal administration when compared with intravenous administration. The absolute bioavailability of a Nanobody or polypeptide to be used in the invention is determined by comparing the concentration vs. time plot of the Nanobody or polypeptide after intravenous (IV) administration with the concentration vs. time plot of the Nanobody or polypeptide after intranasal (IN) administration. The absolute bioavailability of a Nanobody or polypeptide described above the invention is defined as (AUC_{IN} x dose_{IN})/(AUC_{IV} x dose_{IV}) x 100.

The relative bioavailability of the Nanobody or polypeptide to be used in the invention following nasal administration of the composition is of the order of ca. 1, 2, 3, 5, 7, 10, 15, 20, 25, 30, 40, 50, 60, 70, 80,, 90, 100% or more of the levels achieved on injection. Relative bioavailability measures the availability of the active drug in systemic circulation after intranasal administration when compared with another form of administration of the same drug, such as intramuscular (IM) or subcutaneous (SC). The relative bioavailability of a Nanobody or polypeptide is determined by comparing the concentration vs. time plot of the Nanobody or polypeptide after intramuscular (IM) or subcutaneous (SC) administration with the concentration vs. time plot of the Nanobody or polypeptide after intranasal (IN) administration. The relative bioavailability of a Nanobody or polypeptide to be used in the invention is defined as (AUC_{IN} x dose_{IN})/(AUC_{SC/IM} x dose_{SC/IM}) x 100. Accordingly, in order to be equipotent to the injection route, nasal administration will appropriately be effected so as to give a dosage rate of the order of 1 to 100 times, preferably 1 to 50 times, more preferably 1 to 20 times, even more preferably 1 to 10 times the dosage required for treatment via injection, also depending on the frequency of the nasal application.

The amount of active compound will generally be chosen to provide effective treatment on administration once. Alternatively, dosages may be split over a series of e.g. 2 to 4 applications taken at intervals during the day. The sub-dose itself may be further divided, e.g., into a number of discrete loosely spaced administrations; such as multiple inhalations from an insufflator or by application of a plurality of drops. To maintain more consistent or normalized therapeutic levels of the Nanobody or polypeptide, it is advised that the composition to be used in the invention is repeatedly administered to the nasal mucosa of the subject, for example one, two or more times within a 24 hour period, four or more times within a 24 hour period, six or more times within a 24 hour period, or eight or more times within a 24 hour period. Delivery devices are described below that yield pulsatile delivery to maintain normalized and/or elevated therapeutic levels of the Nanobodies, polypeptide or proteins in the blood. An administration regimen could include long-term, daily treatment. By "long-term" is meant at least two weeks and preferably, several weeks, months, or years of duration. The clinician will generally be able to determine a suitable daily dose, depending on the factors mentioned herein. It will also be clear that in specific cases, the clinician may choose to deviate from these amounts, for example on the basis of the factors cited above and his expert judgment.

The final determination of the effective dosage will be based on animal model studies, followed up by human clinical trials, and is guided by determining effective dosages and nasal administration protocols that significantly reduce the occurrence or severity of the targeted disease symptoms or conditions in the subject. Suitable models in this regard include, for example, murine, rat, porcine, feline, non-human primate, and other accepted animal model subjects known in the art. Ultimately, the dosage of Nanobody or polypeptide to be used in the invention will be at the discretion of the attendant, physician or clinician. The dosage can also be adjusted by the individual physician in the event of any complication.

As a non-limiting example, the Nanobody, polypeptide or protein is suitably present in the composition to be used in the invention in an amount such as to provide a free Nanobody, polypeptide or protein concentration from about 0.1 microgram to 0.1 gram per kg body weight per day, such as from 1 microgram to 0.1 gram per kg body weight per day, such as from 0.01 to 100 milligram per kg body weight per day, such as from 0.05-100 milligram, such as from 0.05 to 50 milligram, 0.05 to 30 milligram, 0.1 to 20 milligram, or from about 1 to 10 or about 5 to 10 milligram per kg body weight per day either as a single daily dose or as multiple divided doses during the day.

The proportion of each further component in the nasal composition to be used in the invention may vary depending on the components used. For example, but without being limiting, the amount of nasal carrier may be in the range of from 0.1 to 99.9 % by weight of the total weight or volume of the composition. When present, the amount surfactant may be in the range from about 0.01 to about 10% or higher and preferably about 0.05 to about 1.0% by weight of the total volume or weight of the composition, the amount depending on the specific surfactant used. The amount is generally kept as low as possible since above a certain level no further enhancement of absorption can be achieved and also too high of a surfactant level may cause irritation of the nasal mucosa. The amount of delivery enhancing agents may be at least 0.1%, suitably in the range from about 0.5 to 10 % of the total weight of the composition. Where the composition is liquid, the enhancing agent may suitably be present in an amount of from 0.1 to 5% w/v of the total composition. Preserving agents may be present in an amount of from about 0.002 to 0.02 % by weight of the total weight or volume of the composition.

In addition to the concentration of the different compounds in the composition to be used in the invention, the delivery volume is important to consider as well. The delivery volume is limited by the size of the nasal cavity. Suitable delivery volumes will be clear to a person skilled in the art of pharmacology. Preferably, the total composition quantity administered at each nasal application suitably comprises from about 0.05 to 0.5 ml, preferably about 0.07 to 0.3 ml, typically about 0.09-0.1 ml. A solid composition may comprise from 1 to 30 mg carrier per dosage, more particularly 4 to 20 mg.

The present disclosure further provides a method for the preparation of a composition as described above, comprising bringing a polypeptide or protein comprising or essentially consisting of a Nanobody®, a domain antibody, a single domain antibody or "dAb" and a pharmaceutically acceptable nasal carrier into intimate admixture.

The present disclosure further provides a method for the preparation of a composition as described above, comprising bringing a polypeptide or protein comprising or essentially consisting of a Nanobody®, a domain antibody, a single domain antibody or "dAb" and a pharmaceutically acceptable nasal carrier and, optionally, one or more further therapeutic ingredients and/or pharmaceutical acceptable additives and/or agents into intimate admixture.

The liquid compositions to be used in the invention may be prepared by bringing into intimate admixture the Nanobody, or polypeptide described above in the liquid carrier optionally together with the further ingredients, additives and/or agents. Preferably the resulting mixture is then lyophilized and dissolved in water or aqueous saline for use in a liquid form according to the invention. The solid nasal composition to be used in the invention may be prepared in conventional manner. The Nanobody or polypeptide to be used in the invention may be admixed with the carrier particles, e.g. a polymer base or cellulose product in conventional manner, optionally with further ingredients, additives and/or agents as indicated above e.g. a mucosal delivery enhancing agent or surfactant such as disclosed. The Nanobody, polypeptide or protein may be in solution e.g. an aqueous or alcoholic solution when being mixed with the carrier particles and the solvent evaporated, e.g. under freeze-drying or spray drying. Such drying may be effected under the conventional conditions. Alternatively the mixture may be compacted or granulated and then be pulverized and/or sieved. If desired the particles may be coated. According to a preferred embodiment of the invention, the nasal composition is prepared by lyophilisation. A homogeneous solution, preferably aqueous, containing the Nanobody, polypeptide or protein and optionally containing further ingredients, additives and/or agents as discussed above, is prepared and then submitted to lyophilisation in analogy with known lyophilisation procedures, and to subsequent drying. The resulting powder may then be dissolved in a liquid excipient or nasal carrier before administration, e.g. to reconstitute nasal drops, gel or spray. Alternatively it may be administered as such in the form of lyophilized powder or it may be mixed with further ingredients, additives and/or agents as discussed above. For example, a lyophilized powder comprising the Nanobody, polypeptide or protein but free of any nasal carrier may be prepared and then admixed with the desired nasal carrier or mixture of nasal carriers.

Numerous delivery devices are available for intranasal administration such as instillation catheters, droppers, unit-dose containers, squeeze bottles pump sprays, airless and preservative-fee sprays, compressed air nebulizers, metered-dose inhalers, insufflators and pressurized metered dose inhalers. Devices vary in accuracy of delivery, dose reproducibility, cost, and ease of use. Currently, metered-dose systems provide the greatest dose accuracy and reproducibility. Suitable delivery devices for the composition of the present invention will be clear to a person skilled in the art of pharmacology and are further described herein.

The present invention encompasses the use of any delivery device that is suitable for nasal application and/or administration of the composition as described above, i.e. provided with a means to position the composition as described above to the nasal mucosa. Preferably, such means administers a metered dosage of the composition. The composition to be used in the present invention may be packed in any appropriate form or container as long as a means is provided to deliver (a fixed or metered dose of) the composition to the nasal mucosa.

Such delivery devices are preferably capable of (repeatedly) delivering a unit dose of the composition of the invention, and in particular of (repeatedly) delivering a unit dose of the composition of the invention that essentially comprises a delivery volume that is suitable for nasal administration (i.e. as further described herein).

For administration of a liquid in drop form, for example, such compositions will suitably be put up in a container provided with a conventional dropper/closure device, e.g. comprising a pipette or the like, preferably delivering a substantially fixed volume of composition/drop.

For administration of an aqueous solution as a nasal spray, the aqueous solution may be dispensed in spray form by a variety of methods known to those skilled in the art. For example, such compositions will be put up in an appropriate atomising device, e.g. in a pump-atomiser, or the like. The atomising device will be provided with appropriate means, such as a spray adaptor for delivery of the aqueous spray to the naris. Preferably it will be provided with means ensuring delivery of a substantially fixed volume of composition/actuation (i.e. per spray-unit). Examples of nasal sprays include nasal actuators produced by Ing. Erich Pfeiffer GmbH, Radolfzell, Germany (see US 4,511,069, US 4,778,810, US 5,203,840, US 5,860,567, US 5,893, 484, US 6,227,415, and US 6,364,166. Additional aerosol delivery forms may include, e.g., compressed air-, jet-, ultrasonic-, and piezoelectric nebulizers.

Alternatively the spray may be bottled under pressure in an aerosol device. An "aerosol" is a product that is packaged under pressure and contains therapeutically active ingredients that are released or delivered upon activation of an appropriate valve system. The propellant may be a gas or a liquid (e.g. a fluorinated and/or chlorinated hydrocarbon). The spray composition may be suspended or dissolved in a liquid propellant. Stabilizing and/or suspending agents and/or co-solvents may be present.

A dry powder may be readily dispersed in an inhalation device as described in US 6,514, 496. These dry powder compositions comprise a plurality of discrete dry particles with an average particle size in the range of 0.4 to 5 µm mass median diameter, or in the range of 20 to 250 µm (Garcia-Arieta et al., Biol. Pharm. Bull. 2001; 24: 1411-1416).

If desired a powder or liquid may be filled into a soft or hard capsule or in a single dose device adapted for nasal administration. The powder may be sieved before filled into the capsules such as gelatine capsules. The delivery device may have means to break open the capsule. The powdery nasal composition can be directly used as a powder for a unit dosage form. The contents of the capsule or single dose device may be administered using e.g. an insufflator. Preferably it will be provided with means ensuring dosing of a substantially fixed amount of composition/actuation.

Differences also exist in force of delivery, emitted droplet size, and spray patterns. Delivery devices are important not only for delivering the Nanobody, polypeptide or protein of the invention, but also for providing an appropriate environment for storage. This would include protection from microbial contamination and chemical degradation. The device and formulation should be compatible so as to avoid potential leaching or adsorption.

The container may contain sufficient composition for a single nasal dosages or for the supply of several sequential dosages, e.g. over a period of 1 day, various days or weeks. Suitable quantities of individual dosages are hereinbefore defined. Preferably they enable application of the contained composition in individual fixed quantities of from about 0.05 to 0.15 ml, typically of from about 0.09 to 0.1 ml.

The delivery device may be suitably packaged in a manner known per se. The delivery device (or its packaging) may optionally be provided with a label and/or with instructions for use indicating that the composition should be used intranasally.

In another embodiment, the composition to be used in the invention is provided as a nasal insert having the Nanobody or polypeptide dispersed therein. A "nasal insert" is a device which is sized, shaped and adapted for placement and retention into the naris; or which is intended for insertion into the naris; or which is formed, shaped or otherwise adapted for insertion into and/or retention in the naris; or which is shaped to substantially conform to the internal surface of the naris; or which is provided together with instructions to effect insertion into the naris. The insert may be retained in the naris, but flushed by the nasal mucus, and may be designed to release the Nanobody or polypeptide at the same place in the naris. Suitable nasal insert types include nasal plugs, tampons and the like. Further examples of nasal inserts, their characteristics and preparation are described in EP 490806.

The composition to be used in the invention is formulated for local administration to the nasal mucosa membrane and is capable of providing a systemic therapeutic or biological response of the Nanobody or polypeptide in the subject and/or providing a therapeutic or biological response of the Nanobody or polypeptide in the brain of the subject. This means that there is a sufficient amount of functional (i.e. active or not inactivated) Nanobody, polypeptide or protein present in the blood (and/or another selected physiological compartment, tissue and/or organ such as e.g. the kidney, bladder and/or lung) and/or in the brain to provide the desired therapeutic effect (i.e. to elicit the desired activity or the desired biological, prophylactic or therapeutic response in the subject receiving said Nanobody, polypeptide or protein). The bioavailability of the Nanobody, polypeptide or protein in the blood (and/or another selected physiological compartment, tissue and/or organ such as e.g. the kidney, bladder and/or lung) and/or in the brain following administration of the composition is determined by measuring the pharmacokinetic parameters Cmax (peak concentration), AUC (area under concentration vs. time curve) and/or Tmax (time to maximal blood concentration), which are well known to those skilled in the art (Laursen et al., Eur. J. Endocrinology, 1996; 135: 309- 315). The bioavailability of the Nanobody, peptide or protein may be determined in any conventional manner, e.g. by radioimmunoassay.

"Cmax", as used in the present invention, is the mean maximum concentration of the Nanobody, polypeptide or protein achieved in blood (and/or another selected physiological compartment, tissue and/or organ such as e.g. the kidney, bladder and/or lung) and/or in the brain, following nasal administration of a single dosage of the composition to the subject. Blood or bloodstream as used in the present invention, can be any form and/or fraction of blood. Without being limiting, blood or bloodstream includes plasma and/or serum. The Cmax for the Nanobody, polypeptide or protein comprised in the composition can have any value as long as said Nanobody, polypeptide or protein provides the desired activity or therapeutic or biological response in the subject in need of said Nanobody, polypeptide or protein. In an embodiment of the invention, the Nanobody or polypeptide reaches a Cmax in blood of at least 1 ng of Nanobody or polypeptide per ml of blood. In a further embodiment, the Nanobody or polypeptide reaches a Cmax in blood of at least 2, 5, 10, 15, 20, 30, 40, 50, 100, 150, 200, 300, 400, 500, 750, 100 ng or more of Nanobody or polypeptide per ml of blood.

In another embodiment, the Nanobody, polypeptide or protein reaches a Cmax in blood of at least 1 ng of Nanobody, polypeptide or protein per ml of blood following intranasal administration of a dose of 5 mg/kg body weight of said Nanobody, polypeptide or protein. In a further embodiment, the Nanobody, polypeptide or protein reaches a Cmax in blood of at least 2, 5, 10, 15, 20, 30, 40, 50, 100, 150, 200, 300, 400, 500, 750, 100 ng or more of Nanobody, polypeptide or protein per ml of blood following intranasal administration of a dose of 5 mg/kg body weight of said Nanobody, polypeptide or protein.

In another embodiment of the invention, following intranasal administration of the Nanobody or polypeptide, said Nanobody or polypeptide reaches a Cmax in blood of at least 1% of the Cmax that is reached following parenteral administration of the same amount of Nanobody or polypeptide. In a further embodiment, following intranasal administration of the Nanobody or polypeptide, said Nanobody or polypeptide reaches a Cmax in blood of at least 2, 3, 5, 7, 10, 15, 20, 25, 30, 40, 50% or more of the Cmax that is reached following parenteral administration of the same amount of Nanobody or polypeptide.

In another embodiment of the invention, following intranasal administration of the Nanobody or polypeptide, said Nanobody or polypeptide reaches a Cmax in the brain of at least 1% of the Cmax that is reached following parenteral administration of the same amount of Nanobody or polypeptide. In a further embodiment, following intranasal administration of the Nanobody, polypeptide or protein, said Nanobody, polypeptide or protein reaches a Cmax in the brain of at least 2, 3, 5, 7, 10, 15, 20, 25, 30, 40, 50% or more of the Cmax that is reached following parenteral administration of the same amount of Nanobody, polypeptide or protein.

"Tmax", as used in the present invention, is the mean time to reach maximum concentration of the Nanobody or polypeptide in blood (and/or another selected physiological compartment, tissue and/or organ such as e.g. the kidney, bladder and/or lung) and/or in the brain of the subject, following nasal administration of a single dosage of the composition. The Tmax for the Nanobody or polypeptide comprised in the composition to be used in the invention can have any value as long as said Nanobody or polypeptide provides the desired activity or therapeutic or biological response in the subject in need of said Nanobody or polypeptide. In an embodiment of the invention, the Nanobody or polypeptide reaches the bloodstream with a Tmax of less than 120 minutes. In a further embodiment, the Nanobody or polypeptide reaches the bloodstream with a Tmax of less than 90, 60, 50, 40, 30, 20, 10, or 5 minutes. In another embodiment of the invention, the Nanobody or polypeptide reaches the brain with a Tmax of less than 120 minutes. In a further embodiment, the Nanobody or polypeptide reaches the brain with a Tmax of less than 90, 60, 50, 40, 30, 20, 10, or 5 minutes.

The "concentration vs. time curve" measures the concentration of the Nanobody, polypeptide or protein in blood (and/or another selected physiological compartment, tissue and/or organ such as e.g. the kidney, bladder and/or lung) and/or in the brain of a subject vs. time after administration of a dosage of the composition to be used in the invention.

In an embodiment, the Nanobody or polypeptide to be used in the invention reaches a Cmax in blood of at least 1 ng of Nanobody, polypeptide or protein per ml of blood within less than 120 minutes following intranasal administration of the composition. In a further embodiment, the Nanobody or polypeptide to be used inthe invention reaches a Cmax in blood of at least 2, 5, 10, 15, 20, 30, 40, 50, 100, 150, 200, 300, 400, 500, 750, 1000 ng or more of Nanobody or polypeptide per ml of blood within less than 120 minutes following intranasal administration of the composition. In another embodiment, the Nanobody or polypeptide to be used inthe invention reaches a Cmax in blood of at least 1 ng of Nanobody or polypeptide per ml of blood within less than 90, 60, 50, 40, 30, 20, 10, or 5 minutes following intranasal administration of the composition. In a further embodiment, the Nanobody or polypeptide to be used inthe invention reaches a Cmax in blood of at least 2, 5, 10, 15, 20, 30, 40, 50, 100, 150, 200, 300, 400, 500, 750, 1000 ng or more of Nanobody or polypeptide per ml of blood within less than 90, 60, 50, 40, 30, 20, 10, or 5 minutes following intranasal administration of the composition.

In another embodiment, the Nanobody, polypeptide or protein reaches a Cmax in blood of at least 1 ng of Nanobody, polypeptide or protein per ml of blood within less than 120 minutes following intranasal administration of a dose of 5 mg/kg body weight of said Nanobody, polypeptide or protein. In a further embodiment, the Nanobody, polypeptide or protein reaches a Cmax in blood of at least 2, 5, 10, 15, 20, 30, 40, 50, 100, 150, 200, 300, 400, 500, 750, 1000 ng or more of Nanobody, polypeptide or protein per ml of blood within less than 120 minutes following intranasal administration of a dose of 5 mg/kg body weight of said Nanobody, polypeptide or protein. In another embodiment, the Nanobody, polypeptide or protein reaches a Cmax in blood of at least 1 ng of Nanobody, polypeptide or protein per ml of blood within less than 90, 60, 50, 40, 30, 20, 10, or 5 minutes following intranasal administration of a dose of 5 mg/kg body weight of said Nanobody, polypeptide or protein. In a further embodiment, the Nanobody, polypeptide or protein reaches a Cmax in blood of at least 2, 5, 10, 15, 20, 30, 40, 50, 100, 150, 200, 300, 400, 500, 750, 1000 ng or more of Nanobody, polypeptide or protein per ml of blood within less than 90, 60, 50, 40, 30, 20, 10, or 5 minutes following intranasal administration of a dose of 5 mg/kg body weight of said Nanobody, polypeptide or protein.

The "area under the curve (AUC)", as used in the present invention, is the area under the curve in a plot of concentration of Nanobody, polypeptide or protein in blood (and/or another selected physiological compartment, tissue and/or organ such as e.g. the kidney, bladder and/or lung) and/or in the brain against time. Mathematically, this value is a measure of the integral of the instantaneous concentrations during a time interval. AUC is usually given for the time interval zero to infinity, and other time intervals are indicated (for example AUC (t₁,t₂) where t₁ and t₂ are the starting and finishing times for the interval). Clearly blood (and/or another selected physiological compartment, tissue and/or organ such as e.g. the kidney, bladder and/or lung) and/or brain Nanobody, polypeptide or protein concentrations cannot be measured 'to infinity' for a subject so mathematical approaches are used to estimate the AUC from a limited number of concentration measurements. The AUC (from zero to infinity) is used to measure the total amount of Nanobody, polypeptide or protein absorbed by the body, irrespective of the rate of absorption. This is useful when trying to determine whether two application formulations with the same dose (for example parenteral and intranasal) release the same dose of Nanobody, polypeptide or protein to the body.

The AUC for the Nanobody or polypeptide comprised in the composition to be used in the invention can have any value as long as said Nanobody or polypeptide provides the desired activity or biological response in the subject in need of said Nanobody or polypeptide. In an embodiment of the invention, the AUC for the Nanobody or polypeptide in blood following intranasal administration of a composition comprising said Nanobody or polypeptide is at least 500 ng/ml/minute Nanobody, polypeptide or protein. In a further embodiment, the AUC for the Nanobody or polypeptide in blood following intranasal administration of a composition comprising said Nanobody or polypeptide is at least 600, 700, 800, 900, ng/ml/minute or at least 1, 1.5, 2, 3, 4, 5. 10 or 15 µg/ml/minute Nanobody or polypeptide.

In another embodiment of the invention, the AUC for the Nanobody or polypeptide in blood following intranasal administration of a dose of 5 mg/kg body weight of said Nanobody or polypeptide is at least 500 ng/ml/minute Nanobody or polypeptide. In a further embodiment, the AUC for the Nanobody or polypeptide in blood following intranasal administration of a dose of 5 mg/kg body weight of said Nanobody or polypeptide is at least 600, 700, 800, 900 ng/ml/minute or 1, 1.5, 2, 3, 4, 5 or 10 µg/ml/minute Nanobody or polypeptide per ml of blood.

As discussed above, in an embodiment of the invention, the bioavailability (absolute or relative) for the Nanobody or polypeptide in blood following intranasal administration of a composition comprising said Nanobody or polypeptide is at least 1% compared to parenteral administration of said Nanobody or polypeptide. In a further embodiment, the bioavailability for the Nanobody or polypeptide in blood following intranasal administration of a composition comprising said Nanobody or polypeptide is at least 2, 3, 5, 7, 10, 15, 20, 25, 30, 40, 50, 60, 70, 80, 90, 100% or more compared to parenteral administration of said Nanobody or polypeptide.

In another embodiment of the invention, the bioavailability (absolute or relative) for the Nanobody or polypeptide in the brain following intranasal administration of a composition comprising said Nanobody or polypeptide is at least 1% compared to parenteral administration of said Nanobody or polypeptide. In a further embodiment, the bioavailability for the Nanobody or polypeptide in the brain following intranasal administration of a composition comprising said Nanobody or polypeptide is at least 2, 3, 5, 7, 10, 15, 20, 25, 30, 40, 50, 60, 70, 80, 90, 100% or more compared to parenteral administration of said Nanobody or polypeptide.

Intranasal administration of one or more Nanobodies or polypeptides described above to a subject yields effective delivery of the Nanobodies or polypeptides to the blood (and/or another selected physiological compartment, tissue and/or organ such as e.g. the kidney, bladder and/or lung) and/or to the brain to elicit the desired activity or therapeutic or biological response in the subject. In the present invention, the Nanobody or polypeptide provides the prevention and/or treatment of a selected disease or condition in said subject. Accordingly, another aspect of the invention relates to a medical use in a method for the prevention and/or treatment of a selected disease or condition in a subject in need of a Nanobody or polypeptide as described above, said method comprising nasally administering, to said subject a therapeutically effective amount of said Nanobody or polypeptide, and/or of a composition comprising the same.

In the context of the present invention, the term "prevention and/or treatment" not only comprises preventing and/or treating the disease, but also generally comprises preventing the onset of the disease, slowing or reversing the progress of disease, preventing or slowing the onset of one or more symptoms associated with the disease, reducing and/or alleviating one or more symptoms associated with the disease, reducing the severity and/or the duration of the disease and/or of any symptoms associated therewith and/or preventing a further increase in the severity of the disease and/or of any symptoms associated therewith, preventing, reducing or reversing any physiological damage caused by the disease, and generally any pharmacological action that is beneficial to the patient being treated.

The subject to be treated may be any warm-blooded animal, but is in particular a mammal, and more in particular a human being. As will be clear to the skilled person, the subject to be treated will in particular be a person suffering from, or at risk from, the diseases and/or disorder.

The disclosure also relates to a method for the prevention and/or treatment of at least one disease or disorder that can be prevented and/or treated by administering a Nanobody or polypeptide as described above to a subject suffering from said disease or disorder, said method comprising nasally administering to said subject a therapeutically effective amount of the Nanobody or polypeptide, and/or of a composition comprising the same. Accordingly, the invention relates to the Nanobody or polypeptide as described above for use in the prevention and/or treatment of at least one disease or disorder that can be prevented and/or treated by nasally administering to a subject a Nanobody or polypeptide.

In another embodiment, the disclosure relates to a method for immunotherapy, and in particular for passive immunotherapy, which method comprises intranasal administering, to a subject suffering from or at risk of a diseases and/or disorders that can be cured or alleviated by immunotherapy with a Nanobody or polypeptide as described above, a therapeutically effective amount of said Nanobody or polypeptide and/or of a composition comprising the same.

The Nanobodies and Nanobody-based polypeptides present in the compositions to be used in the invention may be directed against any suitable target that is of therapeutic or diagnostic interest. The Nanobodies and Nanobody-based polypeptides can be functional as agonists as well as antagonists. Examples include but are not limited to targets of therapeutic interests such as TNF-α, IgE, IFN-γ, MMP-12, EGFR, CEA, H. pylori, M. tuberculosis, influenza, β-amyloid, vWF, IL-6, IL-6R, PDK1, CD40, OVA, VSG, S. typhimurium, Rotavirus, Brucella, parathyroid hormone-derived peptides.

The invention provides systemic delivery of the Nanobody or polypeptide as described above. The desired target can be a target in any physiological compartment, tissue or organ. In an embodiment, the Nanobody or polypeptide is directed against a target in the kidney or the bladder and the invention relates to a method for the prevention and/or treatment of a subject in need of a Nanobody or polypeptide that is directed against a target in the kidney or bladder, said method comprising nasally administering, to said subject a therapeutically effective amount of said Nanobody or polypeptide, and/or of a composition comprising the same. The invention also relates to a method for the prevention and/or treatment of at least one disease or disorder that can be prevented and/or treated by administering to a subject suffering from said disease or disorder a Nanobody or polypeptide described above that is directed against a target in the kidney or the bladder, said method comprising nasally administering to said subject a therapeutically effective amount of said Nanobody or polypeptide, and/or of a composition comprising the same. The invention also relates to a method for the prevention and/or treatment of a disease or disorder of the kidney or bladder, said method comprising nasally administering to said subject a therapeutically effective amount of a Nanobody or polypeptide that is directed against a target in the kidney or the bladder and/or of a composition comprising the same. Accordingly, the invention also relates to a composition, wherein the Nanobody or polypeptide as described above is directed against a target in the kidney or the bladder for use in the prevention and/or treatment of a disease or disorder of the kidney or bladder.

In another aspect of the disclosure, the Nanobody, polypeptide or protein is directed against a target in the lung and the disclosure relates to a method for the prevention and/or treatment of a subject in need of a Nanobody, polypeptide or protein that is directed against a target in the lung, said method comprising nasally administering, to said subject a therapeutically effective amount of said Nanobody, polypeptide or protein, and/or of a composition comprising the same. The disclosure also relates to a method for the prevention and/or treatment of at least one disease or disorder that can be prevented and/or treated by administering to a subject suffering from said disease or disorder a Nanobody, polypeptide or protein that is directed against a target in the lung, said method comprising nasally administering to said subject a therapeutically effective amount of said Nanobody, polypeptide or protein, and/or of a composition comprising the same. The disclosure also relates to a method for the prevention and/or treatment of a disease or disorder of the lung, said method comprising nasally administering to said subject a therapeutically effective amount of a Nanobody, polypeptide or protein that is directed against a target in the lung and/or of a composition comprising the same. Accordingly, the disclosure also relates to the composition of the, wherein the Nanobody, polypeptide or protein is directed against a target in the lung for the prevention and/or treatment of at least one disease or disorder of the lung.

In another preferred embodiment, the Nanobody, polypeptide or protein is directed against a target on a tumor cell and the invention relates to a method for the prevention and/or treatment of a subject in need of a Nanobody or polypeptide that is directed against a target on a tumor cell, said method comprising nasally administering, to said subject a therapeutically effective amount of said Nanobody or polypeptide, and/or of a composition comprising the same. The invention also relates to a method for the prevention and/or treatment of at least one disease or disorder that can be prevented and/or treated by administering to a subject suffering from said disease or disorder a Nanobody or polypeptide as described above that is directed against a target on a tumor cell, said method comprising nasally administering to said subject a therapeutically effective amount of said Nanobody or polypeptide, and/or of a composition comprising the same. The invention also relates to a method for the prevention and/or treatment of a tumor related disease or disorder, said method comprising nasally administering to said subject a therapeutically effective amount of a Nanobody or polypeptide that is directed against a target on a tumor and/or of a composition comprising the same. Accordingly, the invention also relates to a composition, wherein the Nanobody or polypeptide is directed against a target on a tumor for use in the prevention and/or treatment of at least one a tumor related disease or disorder.

The disclosure also provides delivery of the Nanobody, polypeptide or protein to the brain. The desired target can be any target in the brain. In an embodiment, the Nanobody, polypeptide or protein is directed against a target in the brain (such as e.g. beta-amyloid) and the disclosure relates to a method for the prevention and/or treatment of a subject in need of a Nanobody, polypeptide or protein that is directed against a target in brain, said method comprising nasally administering, to said subject a therapeutically effective amount of said Nanobody, polypeptide or protein, and/or of a composition comprising the same. The disclosure also relates to a method for the prevention and/or treatment of at least one disease or disorder that can be prevented and/or treated by administering to a subject suffering from said disease or disorder a Nanobody, polypeptide or protein that is directed against a target in the brain, said method comprising nasally administering to said subject a therapeutically effective amount of said Nanobody, polypeptide or protein, and/or of a composition comprising the same. The disclosure also relates to a method for the prevention and/or treatment of a disease or disorder of the brain (such as neurogenetic diseases, (e.g. Huntington's disease and muscular dystrophy), developmental disorders (e.g. cerebral palsy), degenerative diseases of adult life (e.g. Parkinson's disease and Alzheimer's disease), metabolic diseases (e.g. Gaucher's disease), cerebrovascular diseases (e.g. stroke and vascular dementia), trauma (e.g. spinal cord and head injury), convulsive disorders (e.g. Epilepsy) infectious diseases (e.g. AIDS dementia), obesity, diabetes, anorexia, depression, brain tumors, dementia with Lewy bodies, multi-system atrophy, progressive supranuclear palsy, frontotemporal dementia, vascular dementia or Down's syndrome), said method comprising nasally administering to said subject a therapeutically effective amount of a Nanobody, polypeptide or protein that is directed against a target in the brain and/or of a composition comprising the same. Accordingly, the disclosure also relates to the composition, wherein the Nanobody, polypeptide or protein is directed against a target in the brain for the prevention and/or treatment of at least one disease or disorder of the brain (such as neurogenetic diseases, (e.g. Huntington's disease and muscular dystrophy), developmental disorders (e.g. cerebral palsy), degenerative diseases of adult life (e.g. Parkinson's disease and Alzheimer's disease), metabolic diseases (e.g. Gaucher's disease), cerebrovascular diseases (e.g. stroke and vascular dementia), trauma (e.g. spinal cord and head injury), convulsive disorders (e.g. Epilepsy) infectious diseases (e.g. AIDS dementia), obesity, diabetes, anorexia, depression, brain tumors, dementia with Lewy bodies, multi-system atrophy, progressive supranuclear palsy, frontotemporal dementia, vascular dementia or Down's syndrome).

In another embodiment, the Nanobody or polypeptide to be used in the invention is directed against TNF and the invention relates to a method for the prevention and/or treatment of a subject in need of a Nanobody or polypeptide that is directed against TNF, said method comprising nasally administering, to said subject a therapeutically effective amount of said Nanobody or polypeptide, and/or of a composition comprising the same. The invention also relates to a method for the prevention and/or treatment of at least one disease or disorder that can be prevented and/or treated by administering to a subject suffering from said disease or disorder a Nanobody or polypeptide as described above that is directed against TNF, said method comprising nasally administering to said subject a therapeutically effective amount of said Nanobody or polypeptide, and/or of a composition comprising the same. The invention also relates to a method for the prevention and/or treatment of a disease or disorder such as an autoimmune disease (such as e.g. rheumatoid arthritis or Inflammatory Bowel Disease), said method comprising nasally administering to said subject a therapeutically effective amount of a Nanobody or polypeptide that is directed against TNF and/or of a composition comprising the same. Accordingly, the present invention also relates to a composition, wherein the Nanobody or polypeptide is directed against TNF for use in the prevention and/or treatment of at least one disease or disorder such as an autoimmune disease (such as e.g. rheumatoid arthritis or Inflammatory Bowel Disease).

In another embodiment, the Nanobody or polypeptide to be used in the invention is directed against vWF and the invention relates to a method for the prevention and/or treatment of a subject in need of a Nanobody or polypeptide that is directed against vWF, said method comprising nasally administering, to said subject, a therapeutically effective amount of said Nanobody or polypeptide, and/or of a composition comprising the same. The invention also relates to a method for the prevention and/or treatment of at least one disease or disorder that can be prevented and/or treated by administering to a subject suffering from said disease or disorder a Nanobody or polypeptide as described above that is directed against vWF, said method comprising nasally administering to said subject a therapeutically effective amount of said Nanobody or polypeptide, and/or of a composition comprising the same. The invention also relates to a method for the prevention and/or treatment of a disease or disorder related to platelet-mediated aggregation (such as e.g. the formation of a non-occlusive thrombus, the formation of an occlusive thrombus, arterial thrombus formation, acute coronary occlusion, peripheral arterial occlusive disease, restenosis and disorders arising from coronary by-pass graft, coronary artery valve replacement and coronary interventions such angioplasty, stenting or atherectomy, hyperplasia after angioplasty, atherectomy or arterial stenting, occlusive syndrome in a vascular system or lack of patency of diseased arteries, thrombotic thrombocytopenic purpura (TTP), transient cerebral ischemic attack, unstable or stable angina pectoris, cerebral infarction, HELLP syndrome, carotid endarterectomy, carotid artery stenosis, critical limb ischaemia, cardioembolism, peripheral vascular disease, restenosis and myocardial infarction), said method comprising nasally administering to said subject a therapeutically effective amount of a Nanobody or polypeptide that is directed against vWF and/or of a composition comprising the same. Accordingly, the present invention also relates to a composition, wherein the Nanobody or polypeptide is directed against vWF for use in the prevention and/or treatment of at least one disease or disorder related to platelet-mediated aggregation (such as e.g. the formation of a non-occlusive thrombus, the formation of an occlusive thrombus, arterial thrombus formation, acute coronary occlusion, peripheral arterial occlusive disease, restenosis and disorders arising from coronary by-pass graft, coronary artery valve replacement and coronary interventions such angioplasty, stenting or atherectomy, hyperplasia after angioplasty, atherectomy or arterial stenting, occlusive syndrome in a vascular system or lack of patency of diseased arteries, thrombotic thrombocytopenic purpura (TTP), transient cerebral ischemic attack, unstable or stable angina pectoris, cerebral infarction, HELLP syndrome, carotid endarterectomy, carotid artery stenosis, critical limb ischaemia, cardioembolism, peripheral vascular disease, restenosis and myocardial infarction).

In another embodiment, the Nanobody or polypeptide to be used in the invention is directed against IL-6, IL-6R and/or IL-6/IL-6R complex and the invention relates to a method for the prevention and/or treatment of a subject in need of a Nanobody or polypeptide that is directed against IL-6, IL-6R and/or IL-6/IL-6R complex, said method comprising nasally administering, to said subject, a therapeutically effective amount of said Nanobody or polypeptide, and/or of a composition comprising the same. The invention also relates to a method for the prevention and/or treatment of at least one disease or disorder that can be prevented and/or treated by administering to a subject suffering from said disease or disorder a Nanobody or polypeptide as described above that is directed against IL-6, IL-6R and/or IL-6/IL-6R complex, said method comprising nasally administering to said subject a therapeutically effective amount of said Nanobody or polypeptide, and/or of a composition comprising the same. The invention also relates to a method for the prevention and/or treatment of a disease or disorder associated with IL-6R, IL-6 and/or with the IL-6/IL-6R complex (such as e.g. sepsis, various forms of cancer such as multiple myeloma disease (MM), renal cell carcinoma (RCC), plasma cell leukaemia, lymphoma, B-lymphoproliferative disorder (BLPD) and prostate cancer, bone resorption (osteoporosis), cachexia, psoriasis, mesangial proliferative glomerulonephritis, Kaposi's sarcoma, AIDS-related lymphoma, inflammatory diseases and disorder such as rheumatoid arthritis, systemic onset juvenile idiopathic arthritis, hypergammaglobulinemia, Crohn's disease, ulcerative colitis, systemic lupus erythematosus (SLE), multiple sclerosis, Castleman's disease, IgM gammopathy, cardiac myxoma, asthma (in particular allergic asthma) and autoimmune insulin-dependent diabetes mellitus), said method comprising nasally administering to said subject a therapeutically effective amount of a Nanobody or polypeptide that is directed against IL-6, IL-6R and/or IL-6/IL-6R complex and/or of a composition comprising the same. Accordingly, the present invention also relates to a composition, wherein the Nanobody or polypeptide is directed against IL-6, IL-6R and/or IL-6/IL-6R complex for use in the prevention and/or treatment of at least one disease or disorder associated with IL-6R, IL-6 and/or with the IL-6/IL-6R complex (such as e.g. sepsis, various forms of cancer such as multiple myeloma disease (MM), renal cell carcinoma (RCC), plasma cell leukaemia, lymphoma, B-lymphoproliferative disorder (BLPD) and prostate cancer, bone resorption (osteoporosis), cachexia, psoriasis, mesangial proliferative glomerulonephritis, Kaposi's sarcoma, AIDS-related lymphoma, inflammatory diseases and disorder such as rheumatoid arthritis, systemic onset juvenile idiopathic arthritis, hypergammaglobulinemia, Crohn's disease, ulcerative colitis, systemic lupus erythematosus (SLE), multiple sclerosis, Castleman's disease, IgM gammopathy, cardiac myxoma, asthma (in particular allergic asthma) and autoimmune insulin-dependent diabetes mellitus).

Compared to other delivery methods such as oral administration, nasal delivery is an attractive route because of the rapid absorption of the compounds administered and the rapid on set action. Therefore, in a preferred embodiment, the Nanobodies or polypeptides to be used inthe invention or the composition comprising the same is used for the prevention and/or treatment of diseases or disorders which are acute and for which a fast delivery is needed. Accordingly, the invention also relates to a method for the prevention and/or treatment of an acute disorder or disease, said method comprising nasally administering to said subject a therapeutically effective amount of a Nanobody, polypeptide or protein that is capable of alleviating the symptoms of or curing said disorder or disease.

The Nanobodies or polypeptides to be used inthe invention and/or the compositions comprising the same are nasally administered according to a regime of treatment that is suitable for preventing and/or treating the disease or disorder to be prevented or treated. The clinician will generally be able to determine a suitable treatment regimen, depending on factors such as the disease or disorder to be prevented or treated, the severity of the disease to be treated and/or the severity of the symptoms thereof, the specific Nanobody, polypeptide or protein to be used and the pharmaceutical formulation or composition to be used, the age, gender, weight, diet, general condition of the subject, and similar factors well known to the clinician.

Generally, the treatment regimen will comprise the nasal administration of one or more Nanobodies or polypeptides as described above, or of one or more compositions comprising the same, in one or more therapeutically effective amounts or doses. The specific amount(s) or doses to be administered can be determined by the clinician, again based on the factors cited above.

The Nanobodies and polypeptides to be used in the invention may also be used in combination with one or more further therapeutic ingredients (or pharmaceutically active compounds or principles), i.e. as a combined treatment regimen, which may or may not lead to a synergistic effect. Again, the clinician will be able to select such further compounds or principles, as well as a suitable combined treatment regimen, based on the factors cited above and his expert judgement.

When a second active substances or principles is to be used as part of a combined treatment regimen, it can be administered via the same nasal route of administration or via a different route of administration, at essentially the same time or at different times (e.g. essentially simultaneously, consecutively, or according to an alternating regime). When the substances or principles are administered to be simultaneously via the same nasal route of administration, they may be administered as different formulations or compositions or part of a combined formulation or composition, as will be clear to the skilled person.

Also, when two or more active substances or principles are to be used as part of a combined treatment regimen, each of the substances or principles may be administered in the same amount and according to the same regimen as used when the compound or principle is used on its own, and such combined use may or may not lead to a synergistic effect. However, when the combined use of the two or more active substances or principles leads to a synergistic effect, it may also be possible to reduce the amount of one, more or all of the substances or principles to be administered, while still achieving the desired therapeutic action. This may for example be useful for avoiding, limiting or reducing any unwanted side-effects that are associated with the use of one or more of the substances or principles when they are used in their usual amounts, while still obtaining the desired pharmaceutical or therapeutic effect.

The effectiveness of the treatment regimen used according to the invention may be determined and/or followed in any manner known per se for the disease or disorder involved, as will be clear to the clinician. The clinician will also be able, where appropriate and or a case-by-case basis, to change or modify a particular treatment regimen, so as to achieve the desired therapeutic effect, to avoid, limit or reduce unwanted side-effects, and/or to achieve an appropriate balance between achieving the desired therapeutic effect on the one hand and avoiding, limiting or reducing undesired side effects on the other hand.

Generally, the treatment regimen will be followed until the desired therapeutic effect is achieved and/or for as long as the desired therapeutic effect is to be maintained. Again, this can be determined by the clinician.

The invention relates to the use of a Nanobody or polypeptide as described above for the preparation of a medicament for use in a method of treatment comprising systemic delivery of the Nanobody or polypeptide by intranasal administration to the nasal mucosa of a subject. The invention also relates to the use of a Nanobody or polypeptide as described above for the preparation of a composition for the prevention and/or treatment of at least one disease or disorder that can be prevented and/or treated by nasally administering to a subject a Nanobody or polypeptide as described above. The invention also relates to the use of a Nanobody or polypeptide as described above directed against a target in the kidney or the bladder for the preparation of a composition for the prevention and/or treatment of at least one disease or disorder that can be prevented and/or treated by nasally administering to a subject a Nanobody or polypeptide as described above directed against a target in the kidney or the bladder. The invention also relates to the use of a Nanobody or polypeptide as described above directed against a target in the kidney or the bladder for the preparation of a composition for the prevention and/or treatment of at least one disease or disorder of the kidney or bladder. The invention also relates to the use of a Nanobody or polypeptide as described above directed against a target on a tumor for the preparation of a composition for the prevention and/or treatment of at least one disease or disorder that can be prevented and/or treated by nasally administering to a subject a Nanobody or polypeptide as described above directed against a target on a tumor. The invention also relates to the use of a Nanobody or polypeptide as described above directed against a target on a tumor for the preparation of a composition for the prevention and/or treatment of at least one cancer. The invention also relates to the use of a Nanobody or polypeptide as described above directed against TNF for the preparation of a composition for the prevention and/or treatment of at least one disease or disorder that can be prevented and/or treated by nasally administering to a subject a Nanobody or polypeptide as described above directed against TNF. The invention also relates to the use of a Nanobody or polypeptide as described above directed against TNF for the preparation of a composition for the prevention and/or treatment of at least one disease or disorder such as an autoimmune disease (such as e.g. rheumatoid arthritis or Inflammatory Bowel Disease). The invention also relates to the use of a Nanobody or polypeptide as described above directed against vWF for the preparation of a composition for the prevention and/or treatment of at least one disease or disorder that can be prevented and/or treated by nasally administering to a subject a Nanobody or polypeptide as described above directed against vWF. The invention also relates to the use of a Nanobody or polypeptide as described above directed against vWF for the preparation of a composition for the prevention and/or treatment of at least one disease or disorder related to platelet-mediated aggregation (such as e.g. the formation of a non-occlusive thrombus, the formation of an occlusive thrombus, arterial thrombus formation, acute coronary occlusion, peripheral arterial occlusive disease, restenosis and disorders arising from coronary by-pass graft, coronary artery valve replacement and coronary interventions such angioplasty, stenting or atherectomy, hyperplasia after angioplasty, atherectomy or arterial stenting, occlusive syndrome in a vascular system or lack of patency of diseased arteries, thrombotic thrombocytopenic purpura (TTP), transient cerebral ischemic attack, unstable or stable angina pectoris, cerebral infarction, HELLP syndrome, carotid endarterectomy, carotid artery stenosis, critical limb ischaemia, cardioembolism, peripheral vascular disease, restenosis and myocardial infarction). The invention also relates to the use of a Nanobody or polypeptide as described above directed against IL-6, IL-6R and/or IL-6/IL-6R complex for the preparation of a composition for the prevention and/or treatment of at least one disease or disorder that can be prevented and/or treated by nasally administering to a subject a Nanobody or polypeptide as described above directed against IL-6, IL-6R and/or IL-6/IL-6R complex. The invention also relates to the use of a Nanobody or polypeptide as described above directed against IL-6, IL-6R and/or IL-6/IL-6R complex for the preparation of a composition for the prevention and/or treatment of at least one disease or disorder associated with IL-6R, IL-6 and/or with the IL-6/IL-6R complex (such as e.g. sepsis, various forms of cancer such as multiple myeloma disease (MM), renal cell carcinoma (RCC), plasma cell leukaemia, lymphoma, B-lymphoproliferative disorder (BLPD) and prostate cancer, bone resorption (osteoporosis), cachexia, psoriasis, mesangial proliferative glomerulonephritis, Kaposi's sarcoma, AIDS-related lymphoma, inflammatory diseases and disorder such as rheumatoid arthritis, systemic onset juvenile idiopathic arthritis, hypergammaglobulinemia, Crohn's disease, ulcerative colitis, systemic lupus erythematosus (SLE), multiple sclerosis, Castleman's disease, IgM gammopathy, cardiac myxoma, asthma (in particular allergic asthma) and autoimmune insulin-dependent diabetes mellitus).

As discussed above, intranasal administration of one or more Nanobodies or polypeptides as described above to a subject yields effective delivery of the Nanobodies or polypeptides to the blood (and/or another selected physiological compartment, tissue and/or organ such as e.g. the kidney, bladder and/or lung) and/or to the brain to elicit the desired activity or biological response in the subject. In addition to the prophylactic and therapeutic response as discussed above, the Nanobodies or polypeptides may also induce other activities and biological responses. In a preferred embodiment, the present disclosure also provides for the diagnostic use of the Nanobodies, polypeptides and proteins, e.g. for in situ or in vivo labeling, such as radiolabeling and imaging. The present disclosure, therefore, also relates to a diagnostic method comprising the step of nasally administering the Nanobodies, polypeptides or proteins and/or a composition comprising the same. In an aspect of the disclosure, a diagnostic method is provided comprising the steps of nasally administering the Nanobodies, polypeptides or proteins and/or a composition comprising the same and in situ detecting said Nanobodies, polypeptides or proteins. Detection may be done by any method known in the art.

The Nanobodies, polypeptides and proteins can be determined *in situ* by non-invasive methods including but not limited to SPECT and PET, or imaging methods described by Cortez-Retamozo V. (Nanobodies: single domain antibody fragments as imaging agents and modular building blocks for therapeutics, PhD Dissertation, Vrije Universiteit Brussel, Belgium, June 2004), Arbit et al. (Eur. J. Nucl. Med. 1995; 22: 419-426.), Tamada et al. (Microbiol-Immunol. 1995; 39: 861-871), Wakabayashi et al. (Noshuyo-Byori 1995; 12: 105-110), Huang et al. (Clin. Med. J. 1996; 109: 93-96), Sandrock et al. (Nucl. Med. Commun. 1996; 17: 311-316), and Mariani et al. (Cancer 1997; 15: 2484-2489). These *in vivo* imaging methods may allow the localization and possibly quantification a certain target, for example, by use of a labeled Nanobody, polypeptide or protein, specifically recognizing said target. *In vivo* multiphoton microscopy (Bacskai et al., J. Cereb. Blood Flow Metab. 2001; 22: 1035-1041) can be used to image the presence of a certain target with labeled Nanobodies, polypeptides or proteins specific for the target.

The Nanobody, polypeptide or protein nasally administered in the diagnostic methods may be labeled by an appropriate label. The particular label or detectable group used in the method is not a critical aspect of the disclosure, so long as it does not significantly interfere with the specific binding of the Nanobody, polypeptide or protein used in the method. The detectable group can be any material having a detectable physical or chemical property. Such detectable labels have been well developed in the field of immunoassays and, in general, almost any label useful in such methods can be applied to the method of the present invention. Thus, a label is any composition detectable by spectroscopic, photochemical, biochemical, immunochemical, electrical, optical, radiological or chemical means. Useful labels include but are not limited to magnetic beads (e.g. Dynabeads™), fluorescent dyes (e.g. fluorescein isothiocyanate, Texas red, rhodamine, Cy3, Cy5, Cy5.5, Alexi 647 and derivatives), radiolabels (e.g. ³H, ¹²⁵I, ³⁵S, ¹⁴C, ³²P or ^{99m}Tc), enzymes (e.g. horseradish peroxidase, alkaline phosphatase and others commonly used in an ELISA), and colorimetric labels such as colloidal gold, colored glass or plastic (e.g. polystyrene, polypropylene, latex, etc.) beads.

The label may be coupled directly or indirectly to the Nanobody, polypeptide or protein according to methods well known in the art. As indicated above, a wide variety of labels may be used, with the choice of label depending on the sensitivity required, the ease of conjugation with the compound, stability requirements, the available instrumentation and disposal provisions. Non-radioactive labels are often attached by indirect means. Means for detecting labels are well known in the art. Thus, for example, where the label is a radioactive label, means for detection include a scintillation counter or photographic film as in autoradiography. Where the label is a fluorescent label, it may be detected by exciting the fluorophore with the appropriate wavelength of light and detecting the resulting fluorescence. The fluorescence may be detected visually, by means of a photographic film, by the use of electronic detectors such as charge coupled devices (CCDs) or photomultipliers and the like.

The invention will now be further described by means of the following non-limiting examples and figures.

### EXAMPLES

### Example 1: Intranasal administration of the Nanobody FC44 resulted in systemic delivery

To evaluate whether intranasal administration of Nanobodies results in systemic delivery, the biodistribution of the radiolabeled Nanobody FC44 was determined following intranasal delivery. The Nanobody was site-specific radiolabeled at the his-tag with ^{99m}Tc tricarbonyl. In short, the FC44 Nanobody was purified using IMAC (immobilized metal affinity chromatography), size exclusion chromatography and anion exchange chromatography. The lipopolysaccharide (LPS) levels were determined by a quantitative chromogenic limulus amebocyte lysate assay (BioWhittaker/Cambrex, Verviers, Belgium) and were found to be below 5EU/mg protein.

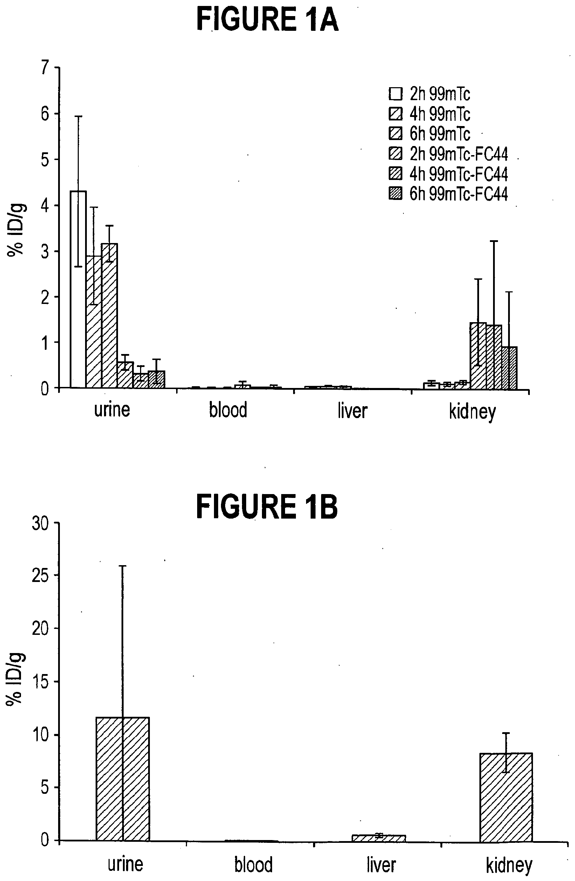

^{99m}Tc tricarbonyl ([^{99m}Tc(CO)3(OH2)3]) was prepared using the commercially available Isolink kit (Mallinckrodt, Hazelwood, MO, USA). 100µl FC44 (1.1mg/ml) was added to 400µl of ^{99m}Tc(CO)3(OH2)3 (30-40mCi) and 100µl carbonate buffer, and incubated at 52°C during 60 minutes. The labeling efficiency as determined via thin layer chromatography was above 90%. Once the quality was confirmed, the radiolabeled Nanobodies were immediately used for in vivo experiments. Male Wistar rats (between 300g-400g) (n=5) were treated intranasal with either free ^{99m}Tc tricarbonyl or ^{99m}Tc-FC44 and the biodistribution of the label was determined after 2, 4 and 6 hours. At time point 0, the rats were anesthetized with fluorane and 50 µl of free radiolabel or radiolabeled FC44 was applied to each nostril of the rat. At the indicated time, rats were killed using pentobarbital sodium and tissue samples were collected (urine, blood, liver, kidneys) and weighed. In parallel, rats (n=2) were injected intravenously with ^{99m}Tc-FC44 and the same tissue samples were collected at 2h. ^{99m}Tc levels were detected using a gamma counter and the resulting counts were corrected for radioactive decay. Results are represented as % injected dose/ gram tissue (%ID/g).
As shown in Figure 1A, ^{99m}Tc-FC44 showed a strong accumulation in the kidneys, while lower levels were observed in urine. Moreover, the higher and respectively lower levels of free ^{99m}Tc-tricarbonyl in urine and kidneys, indicate that the signal observed for ^{99m}Tc-FC44 was originating from radiolabeled Nanobody, rather then from free radiolabel. Since Nanobodies in circulation are primarily cleared through the kidneys and the urine and since the only transport between the nasal cavity and the kidneys is possible via the circulation, these results show that Nanobodies were delivered systemically after intranasal administration. In addition, 2h after administration only slightly higher Nanobody levels were detected in the kidney for the iv injection compared to the intranasal delivery (Figures 1A and 1B).
In conclusion, intranasal administration of the FC44 Nanobody resulted in high kidney and urine levels, underscoring the systemic delivery.

### Example 2: The FC44 Nanobody was still intact after intranasal delivery

The FC44 Nanobody was purified and ^{99m}Tc radiolabeled as described in example 1. A male Wistar rat was treated intranasal with ^{99m}Tc-FC44 as described in example 1 and 4h later the kidneys and the urine were collected. A kidney homogenate was prepared in physiological serum using a homogenizer. Both samples were analyzed on 15% SDS polyacrylamide gel electrophoresis (SDS PAGE). The ^{99m}Tc radiolabel was visualized using Single Photon Emission Computed Tomography (SPECT: Siemens ECAM camera, dual head). Figure 2 clearly shows that in the urine a signal (lane 2) was detected at the same size as the FC44 Nanobody (lane 1), showing that at least a fraction of the Nanobody was still intact after intranasal administration and the resulting systemic delivery. In addition, a signal was detected in the kidney sample (lane 3) although the detected band was more diffuse compared to the urine signal. This was due to the higher viscosity of the kidney sample.
In conclusion, intact Nanobodies can be detected in urine and kidney after intranasal delivery.

### Example 3: The FC44 Nanobody was detected in the circulation

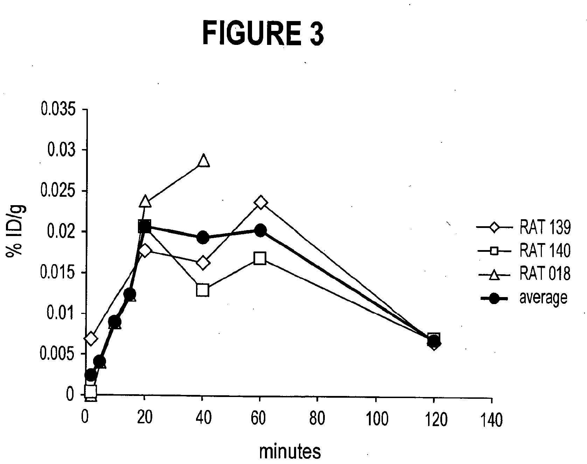

The FC44 Nanobody was purified and ^{99m}Tc radiolabeled as described in example 1. Intranasal and intravenous administration of ^{99m}Tc-FC44 in male Wistar rats was performed as described in example 1. Blood samples of two rats (No. 139 and No. 140) were harvested at 0, 20, 40, 60 and 120 minutes. For another rat (No. 018), blood samples were taken at 0, 5, 10, 15, 20 and 40 minutes. Blood samples were counted in a gamma counter and the resulting counts were corrected for radioactive decay. Results of the intranasal administration are represented as % injected dose/ gram tissue (%ID/g). Whenever possible the average of the ^{99m}Tc signal at 1 time point was calculated. This average %ID/g and the individual % ID/g values are depicted in Figure 3. The maximal Nanobody level was 0.021% ID/g (Cmax) and was reached after 20 minutes (Tmax). During 40 minutes a steady state level of Nanobodies was observed in the circulation, after which the levels started to decrease. Extrapolation of the value of 0.021% ID/g suggests a Cmax of ± 14.6 ng/ml at a dose of 0.1.-0.3 mg/kg and an AUC₀₋₂ₕ of 1258 ng/ml/minute. The absolute bioavailability compared to intravenous injection was 7.4%.
In conclusion, intranasal administration of a short lived monovalent Nanobody resulted in circulating levels in the ng/ml range.

### Example 4: Multiple Nanobodies were delivered systemically after intranasal administration and were still functional

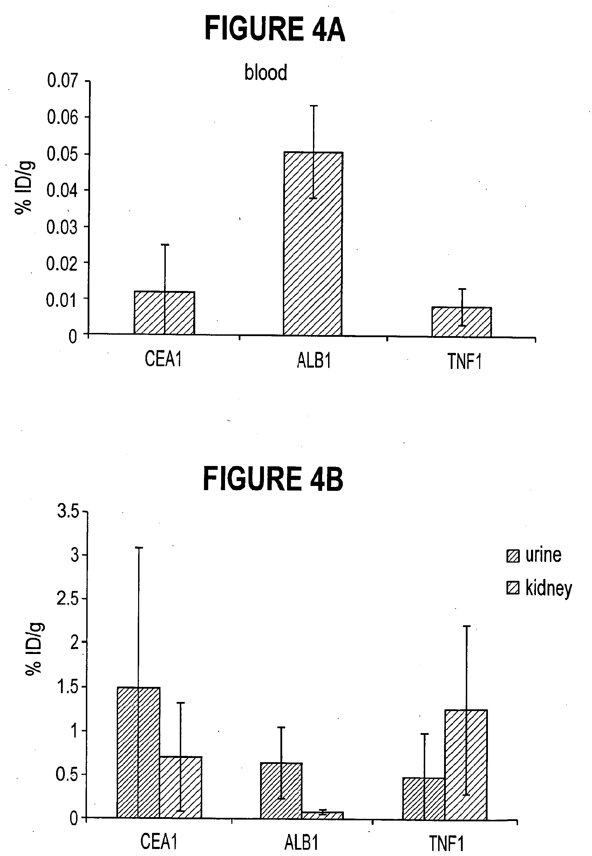

Multiple Nanobodies targeting human CEA (CEA1: 8.56 mg/ml), human TNF (TNF1: 4 mg/ml) and human albumin (ALB1: 4 mg/ml) were purified and ^{99m}Tc radiolabeled as described in example 1. The different Nanobodies were administered intranasally to male Wistar rats as described in example 1. After 4h, blood, urine and kidneys were collected as described in example 1. Samples were counted in a gamma counter and the radioactive signal was corrected for radioactive decay. Data are represented as %ID/g. Figure 4A shows levels of Nanobodies in blood; Figure 4B shows levels of Nanobodies in urine and kidney.
All Nanobodies could be detected in either the kidney, urine and/or blood, underscoring the systemic delivery of the Nanobodies. Moreover, ALB1 showed at least five fold higher blood levels compared to the other Nanobodies. Since the ALB1 Nanobody also interacts with rat albumin in circulation, the Nanobody adopted the albumin half life resulting in a longer half life in comparison to other monovalent Nanobodies like CEA1 and TNF1. This shows that Nanobodies after intranasal administration were still functional in circulation and that the intranasal administration route of Nanobodies can be of therapeutic significance.

### Example 5: Intranasal administration of CEA1 Nanobody resulted in the targeting of CEA positive tumors.

Intravenous delivery of the CEA1 Nanobody, which binds specifically to the CEA (Carcinoembryonic antigen) tumor marker, has been shown to result in CEA positive tumor imaging (Cortez-Retamozo V., Nanobodies: single domain antibody fragments as imaging agents and modular building blocks for therapeutics, PhD Dissertation, Vrije Universiteit Brussel, Belgium, June 2004). It was evaluated whether intranasal delivery of CEA1 also results in CEA positive tumor imaging.
Nude rats (Nu/Nu) were grafted on each thigh with either LS174T or A431 cells. LS174T tumor cells express the CEA antigen, while the A431 cell line is CEA negative. Approximately 10 days later, when tumors were clearly visible, the tumor targeting of the CEA1 Nanobody was evaluated. The CEA1 Nanobody was produced, purified and radiolabeled as described in example 1. Respectively 3 and 5 tumor bearing rats were treated iv and intranasally with ^{99m}Tc-CEA1. Tumor targeting was determined by isolating the tumor tissues 3h after administration. ^{99m}Tc levels in the tumors were determined via gamma counting and the resulting counts were corrected for radioactive decay. Results are represented as % injected dose/ gram tissue (%ID/g).
Table 1 shows that iv injection of CEA1 resulted in a 5-fold specific targeting of the LS174T tumor compared to the A431 tumor (ratio LS174T/A431). Similarly, intranasal delivery of CEA1 led to 2.5 fold specific accumulation, underscoring again the delivery of functional Nanobody to the circulation after intranasal delivery.

**Table 1: CEA tumor specific accumulation of the CEA1 Nanobody. IN: intranasal delivery, IV: intravenous delivery**

| | **AVERAGE** | | **STDEV** | | **ratio LS174T/A431** | |
|---|---|---|---|---|---|---|
| | **% ID/g (IN)** | **% ID/g (IV)** | **% ID/g (IN)** | **% ID/g (IV)** | **(IN)** | **(IV)** |
| **LS174T** | 0.00303903 | 0.47306664 | 0.001527989 | 0.049031221 | 2.619905326 | 5.128418734 |
| **A431** | 0.001159977 | 0.092244153 | 0.00033763 | 0.0258594 | | |

### Example 6: Comparison oral delivery versus intranasal delivery

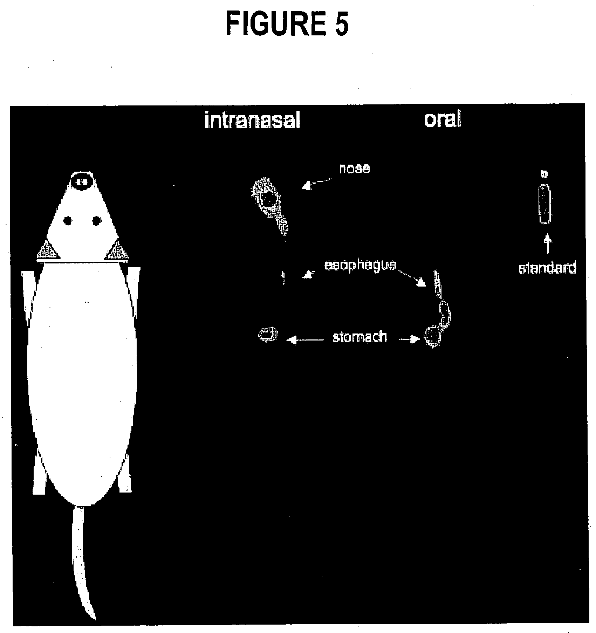

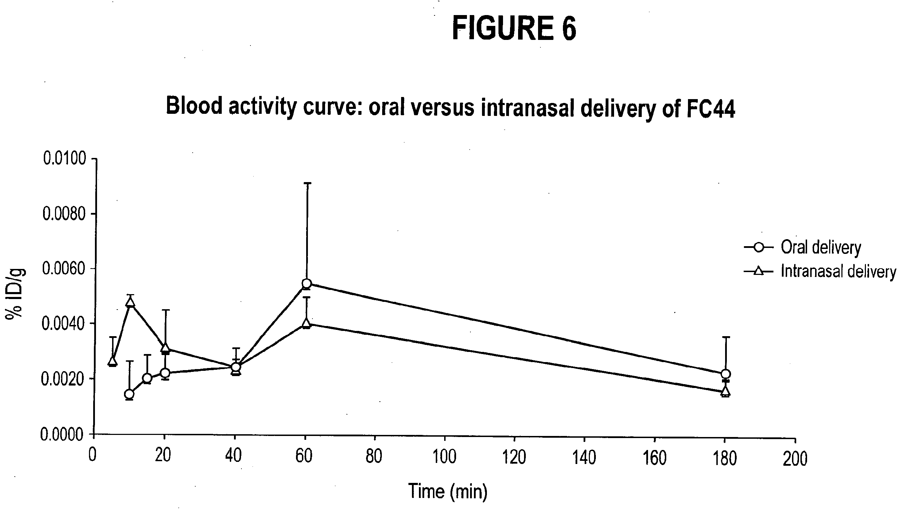

In order to check whether the observed PK profile (see example 3) is due to intranasal or oral absorption, FC44 was applied following these 2 routes.
The FC44 Nanobody was purified and ^{99m}Tc radiolabeled as described in example 1. Male ^{99m}Tc-FC44 was administered to Wistar rats via the intranasal route (n=5) as described in example 1 or via the oral route (n=5) using gastric gavage. Planar gamma camera imaging of rats indicated that the radiolabeled Nanobody indeed localized to the respective site of administration: intranasal delivery of FC44 results in the distribution to the nasal cavity, the esophagus and the stomach; oral delivery mainly results in distribution to the lower esophagus and the stomach (Figure 5). Blood samples were collected 1h and 3h post administration and counted in a gamma counter. The resulting counts were corrected for radioactive decay. Results are represented as % injected dose/ gram tissue (%ID/g). The blood activity curve of intranasal delivery shows an early peak (10-15 min) and a late peak (60 min) (Figure 6). The curve for the oral delivery shows only a late peak (60 min) which coincides with the late peak from the intranasal delivery. This suggests that the late peak observed after intranasal delivery can be attributed to the unwanted gastro-intestinal localization and resulting gastro-intestinal absorption of radiolabeled Nanobody. This underscores that the early peak observed at 10-15 minutes is due to intranasal absorption of FC44.
In conclusion, Nanobodies can be delivered systemically through rapid intranasal absorption.

The terms and expressions which have been employed are used as terms of description and not of limitation, and there is no intention in the use of such terms and expressions of excluding any equivalents of the features shown and described or portions thereof, it being recognized that various modifications are possible within the scope of this embodiment.

## Claims

1. Composition which comprises a therapeutically effective amount of
(i) a polypeptide, which polypeptide consists of one or two Nanobodies, domain antibodies, single domain antibodies or dAbs, optionally linked via a linker,
(ii) and a pharmaceutically acceptable nasal carrier, for use in a method of treatment comprising inducing systemic delivery of the polypeptide by intranasal administration to the nasal mucosa of a subject.

2. The composition for use according to claim 1, wherein said Nanobody, domain antibody, single domain antibody or dAb is directed against a target in the kidney or the bladder, for the prevention and/or treatment of a disease or disorder of the kidney or bladder.

3. The composition for use according to claim 1, wherein said Nanobody, domain antibody, single domain antibody or dAb is directed against a target on a tumor, for the prevention and/or treatment of at least one tumor related disease or disorder.

4. The composition for use according to claim 1, wherein said Nanobody, domain antibody, single domain antibody or dAb is directed against TNF, for the prevention and/or treatment of at least one disease or disorder such as an autoimmune disease, wherein the autoimmune disease optionally is rheumatoid arthritis or Inflammatory Bowel Disease.

5. The composition for use according to claim 1, wherein said Nanobody, domain antibody, single domain antibody or dAb is directed against vWF for use in a method of prevention and/or treatment of the formation of a non-occlusive thrombus, the formation of an occlusive thrombus, arterial thrombus formation, acute coronary occlusion, peripheral arterial occlusive disease, restenosis and disorders arising from coronary by-pass graft, coronary artery valve replacement and coronary interventions such angioplasty, stenting or atherectomy, hyperplasia after angioplasty, atherectomy or arterial stenting, occlusive syndrome in a vascular system or lack of patency of diseased arteries, thrombotic thrombocytopenic purpura (TTP), transient cerebral ischemic attack, unstable or stable angina pectoris, cerebral infarction, HELLP syndrome, carotid endarterectomy, carotid artery stenosis, critical limb ischaemia, cardioembolism, peripheral vascular disease, restenosis and/or myocardial infarction.

6. The composition for use according to claim 1, wherein said Nanobody, domain antibody, single domain antibody or dAb is directed against IL-6, IL-6R and/or IL-6/IL-6R complex for use in a method of prevention and/or treatment of the formation of sepsis, various forms of cancer such as multiple myeloma disease (MM), renal cell carcinoma (RCC), plasma cell leukaemia, lymphoma, B-lymphoproliferative disorder (BLPD) and prostate cancer, bone resorption (osteoporosis), cachexia, psoriasis, mesangial proliferative glomerulonephritis, Kaposi's sarcoma, AIDS-related lymphoma, inflammatory diseases and disorder such as rheumatoid arthritis, systemic onset juvenile idiopathic arthritis, hypergammaglobulinemia, Crohn's disease, ulcerative colitis, systemic lupus erythematosus (SLE), multiple sclerosis, Castleman's disease, IgM gammopathy, cardiac myxoma, asthma, in particular allergic asthma and/or autoimmune insulin-dependent diabetes mellitus.

7. The composition for use according to any one of claims 1 to 6, wherein said Nanobody, domain antibody, single domain antibody or dAb is a humanized V_{HH} or a camelized V_{H}.

8. The composition for use according to any one of claims 1 to 7, wherein the nasal carrier is liquid, semi-liquid, a gel, a cream, a paste, a powder or solid, or may be a film or an encapsulation material.

9. The composition for use according to any one of claims 1 to 8, additionally comprising one or more further therapeutic ingredients.

10. The composition for use according to any one of claims 1 to 9, which is comprised in a delivery device for intranasal administration, which is provided with means enabling the application of said composition to the nasal mucosa.

11. The composition for use according to claim 10, wherein said means enabling the application of said composition to the nasal mucosa is selected from the group of delivery devices consisting of:
- a container provided with a conventional dropper/closure device;
- a squeeze bottle pump spray;
- an airless and preservative-free spray;
- a nasal insert.

12. The composition for use according to claim 11, wherein said delivery device is a metered dose system.

13. Use of
(i) a polypeptide, which polypeptide consists of one or two
Nanobodies, domain antibodies, single domain antibodies or dAbs, optionally linked via a linker,
(ii) and a pharmaceutically acceptable nasal carrier, for the preparation of a medicament which comprises a therapeutically effective amount of the polypeptide, for use in a method of treatment comprising systemic delivery of the polypeptide by intranasal administration to the nasal mucosa of a subject.

14. The use according to claim 13, wherein said Nanobody, domain antibody, single domain antibody or dAb is directed against a target in the kidney or the bladder, for the preparation of a medicament for the prevention and/or treatment of a disease or disorder of the kidney or bladder.

15. The use according to claim 13, wherein said Nanobody, domain antibody, single domain antibody or dAb is directed against a target on a tumor, for the preparation of a medicament for the prevention and/or treatment of at least one tumor related disease or disorder.

16. The use according to claim 13, wherein said Nanobody, domain antibody, single domain antibody or dAb is directed against TNF, for the preparation of a medicament for the prevention and/or treatment of at least one disease or disorder such as an autoimmune disease, wherein the autoimmune disease optionally is rheumatoid arthritis or Inflammatory Bowel Disease.

17. The use according to claim 13, wherein said Nanobody, domain antibody, single domain antibody or dAb is directed against vWF, for the preparation of a medicament for the prevention and/or treatment of the formation of a non-occlusive thrombus, the formation of an occlusive thrombus, arterial thrombus formation, acute coronary occlusion, peripheral arterial occlusive disease, restenosis and disorders arising from coronary by-pass graft, coronary artery valve replacement and coronary interventions such angioplasty, stenting or atherectomy, hyperplasia after angioplasty, atherectomy or arterial stenting, occlusive syndrome in a vascular system or lack of patency of diseased arteries, thrombotic thrombocytopenic purpura (TTP), transient cerebral ischemic attack, unstable or stable angina pectoris, cerebral infarction, HELLP syndrome, carotid endarterectomy, carotid artery stenosis, critical limb ischaemia, cardioembolism, peripheral vascular disease, restenosis and/or myocardial infarction.

18. The use according to claim 13, wherein said Nanobody, domain antibody, single domain antibody or dAb is directed against IL-6, IL-6R and/or IL-6/IL-6R complex, for the preparation of a medicament for the prevention and/or treatment of the formation of sepsis, various forms of cancer such as multiple myeloma disease (MM), renal cell carcinoma (RCC), plasma cell leukaemia, lymphoma, B-lymphoproliferative disorder (BLPD) and prostate cancer, bone resorption (osteoporosis), cachexia, psoriasis, mesangial proliferative glomerulonephritis, Kaposi's sarcoma, AIDS-related lymphoma, inflammatory diseases and disorder such as rheumatoid arthritis, systemic onset juvenile idiopathic arthritis, hypergammaglobulinemia, Crohn's disease, ulcerative colitis, systemic lupus erythematosus (SLE), multiple sclerosis, Castleman's disease, IgM gammopathy, cardiac myxoma, asthma (in particular allergic asthma) and/or autoimmune insulin-dependent diabetes mellitus.

## Patentansprüche

1. Zusammensetzung, umfassend eine therapeutisch wirksame Menge
(i) eines Polypeptids, wobei das Polypeptid aus einem oder zwei Nanokörpern (Nanobodies), Domänen-Antikörpern, Einzeldomänen-Antikörpern oder dAbs besteht, optional verknüpft durch einen Linker,
(ii) und einem pharmazeutisch akzeptablen nasalen Träger
zur Verwendung in einem Verfahren zur Behandlung, umfassend das Induzieren der systemischen Zuführung des Polypeptids durch intranasale Verabreichung an die Nasenschleimhaut eines Subjekts.

2. Zusammensetzung zur Verwendung gemäß Anspruch 1, wobei der Nanokörper, Domänen-Antikörper, Einzeldomänen-Antikörper oder dAb gegen ein Ziel in der Niere oder der Blase gerichtet ist, zur Prävention und/oder Behandlung einer Erkrankung oder Störung der Niere oder Blase.

3. Zusammensetzung zur Verwendung gemäß Anspruch 1, wobei der Nanokörper, Domänen-Antikörper, Einzeldomänen-Antikörper oder dAb gegen ein Ziel auf einem Tumor gerichtet ist, zur Prävention und/oder Behandlung wenigstens einer mit einem Tumor in Zusammenhang stehenden Erkrankung oder Störung.

4. Zusammensetzung zur Verwendung gemäß Anspruch 1, wobei der Nanokörper, Domänen-Antikörper, Einzeldomänen-Antikörper oder dAb gegen TNF gerichtet ist, zur Prävention und/oder Behandlung wenigstens einer Erkrankung oder Störung, wie beispielsweise einer Autoimmunerkrankung, wobei die Autoimmunerkrankung optional rheumatoide Arthritis oder entzündliche Darmerkrankung ist.

5. Zusammensetzung zur Verwendung gemäß Anspruch 1, wobei der Nanokörper, Domänen-Antikörper, Einzeldomänen-Antikörper oder dAb gegen vWF gerichtet ist, zur Verwendung in einem Verfahren zur Prävention und/oder Behandlung der Bildung eines nicht-okklusiven Thrombus, der Bildung eines okklusiven Thrombus, der Bildung eines arteriellen Thrombus, eines akuten Koronarverschlusses, peripherer arterieller Verschlusskrankheit, Restenose und Störungen, verursacht durch koronare Bypässe, Koronararterien-Klappenersatz und koronare Interventionen, wie beispielsweise Angioplastie, Stenting oder Atherektomie, Hyperplasie nach Angioplastie, Atherektomie oder arteriellem Stenting, Verschlusssyndrom in einem Gefäßsystem oder fehlende Durchgängigkeit erkrankter Arterien (lack of patency of diseased arteries), thrombotischer thrombozytopenischer Purpura (TTP), transitorischer ischämischer Hirnattacke, instabiler oder stabiler Angina pectoris, Hirninfarkt, HELLP-Syndrom, Karotis-Endarterektomie, Verengung der Halsschlagader, kritischer Ischämie der Gliedmaßen (critical limb ischaemia), kardialer Embolie (cardioembolism), peripherer Gefäßerkrankung, Restenose und/oder Myokardinfarkt.

6. Zusammensetzung zur Verwendung gemäß Anspruch 1, wobei der Nanokörper, Domänen-Antikörper, Einzeldomänen-Antikörper oder dAb gegen IL-6, IL-6R und/oder IL-6/IL-6R-Komplex gerichtet ist, zur Verwendung in einem Verfahren zur Prävention und/oder Behandlung der Bildung von Sepsis, verschiedener Formen von Krebs, wie beispielsweise Multiplem Myelom (MM), Nierenzellkarzinom (renal cell carcinoma; RCC), Plasmazell-Leukämie, Lymphom, B-lymphoproliferativer Störung (B-lymphoproliferative disorder; BLPD) und Prostatakrebs, Knochenresorption (Osteoporose), Kachexie, Psoriasis, mesangialer proliferativer Glomerulonephritis, Kaposi-Sarkom, AIDS-verwandtem Lymphom, inflammatorischer Erkrankungen und Störungen, wie beispielsweise rheumatoider Arthritis, systemischer juveniler idiopathischer Arthritis, Hypergammaglobulinämie, Morbus Crohn, ulzerativer Colitis, systemischem Lupus erythematodes (SLE), Multipler Sklerose, Castleman-Erkrankung, IgM-Gammopathie, kardialem Myxom, Asthma, insbesondere allergischem Asthma und/oder autoimmunem insulinpflichtigem Diabetes mellitus.

7. Zusammensetzung zur Verwendung gemäß irgendeinem der Ansprüche 1 bis 6, wobei der Nanokörper, Domänen-Antikörper, Einzeldomänen-Antikörper oder dAb eine humanisierte V_{HH} oder eine kamelisierte V_{H} ist.

8. Zusammensetzung zur Verwendung gemäß irgendeinem der Ansprüche 1 bis 7, wobei der nasale Träger flüssig, halbflüssig, ein Gel, eine Creme, eine Paste, ein Pulver oder ein Feststoff ist oder ein Film oder ein Verkapselungsmaterial sein kann.

9. Zusammensetzung zur Verwendung gemäß irgendeinem der Ansprüche 1 bis 8, die zusätzlich einen oder mehrere therapeutische Inhaltsstoffe umfasst.

10. Zusammensetzung zur Verwendung gemäß irgendeinem der Ansprüche 1 bis 9, die in einer Zuführungsvorrichtung für intranasale Verabreichung enthalten ist, die mit Mitteln versehen ist, welche die Anwendung der Zusammensetzung auf der Nasenschleimhaut ermöglichen.

11. Zusammensetzung zur Verwendung gemäß Anspruch 10, wobei das Mittel, welches die Anwendung der Zusammensetzung auf der Nasenschleimhaut ermöglicht, aus der Gruppe von Zuführungsvorrichtungen ausgewählt ist, bestehend aus:
- einem mit einer herkömmlichen Tropfer-/Verschlussvorrichtung versehen Behälter;
- einem Quetschflaschen-Pumpspray;
- einem luftlosen und konservierungsmittelfreien Spray;
- einem Naseneinsatz.

12. Zusammensetzung zur Verwendung gemäß Anspruch 11, wobei die Zuführungsvorrichtung ein System mit abgemessenen Dosen ist.

13. Verwendung
(i) eines Polypeptids, wobei das Polypeptid aus einem oder zwei Nanokörpern, Domänen-Antikörpern, Einzeldomänen-Antikörpern oder dAbs besteht, optional verknüpft durch einen Linker,
(ii) und eines pharmazeutisch akzeptablen nasalen Trägers für die Herstellung eines Medikaments, welches eine therapeutisch wirksame Menge des Polypeptids umfasst, zur Verwendung in einem Verfahren zur Behandlung, umfassend die systemische Zuführung des Polypeptids durch intranasale Verabreichung an die Nasenschleimhaut eines Subjekts.

14. Verwendung gemäß Anspruch 13, wobei der Nanokörper, Domänen-Antikörper, Einzeldomänen-Antikörper oder dAb gegen ein Ziel in der Niere oder der Blase gerichtet ist, für die Herstellung eines Medikaments zur Prävention und/oder Behandlung einer Erkrankung oder Störung der Niere oder Blase.

15. Verwendung gemäß Anspruch 13, wobei der Nanokörper, Domänen-Antikörper, Einzeldomänen-Antikörper oder dAb gegen ein Ziel auf einem Tumor gerichtet ist, für die Herstellung eines Medikaments zur Prävention und/oder Behandlung wenigstens einer mit einem Tumor in Zusammenhang stehenden Erkrankung oder Störung.

16. Verwendung gemäß Anspruch 13, wobei der Nanokörper, Domänen-Antikörper, Einzeldomänen-Antikörper oder dAb gegen TNF gerichtet ist, für die Herstellung eines Medikaments zur Prävention und/oder Behandlung wenigstens einer Erkrankung oder Störung, wie beispielsweise einer Autoimmunerkrankung, wobei die Autoimmunerkrankung optional rheumatoide Arthritis oder entzündliche Darmerkrankung ist.

17. Verwendung gemäß Anspruch 13, wobei der Nanokörper, Domänen-Antikörper, Einzeldomänen-Antikörper oder dAb gegen vWF gerichtet ist, für die Herstellung eines Medikaments zur Prävention und/oder Behandlung der Bildung eines nicht-okklusiven Thrombus, der Bildung eines okklusiven Thrombus, der Bildung eines arteriellen Thrombus, eines akuten Koronarverschlusses, peripherer arterieller Verschlusskrankheit, Restenose und Störungen, verursacht durch koronare Bypässe, Koronararterien-Klappenersatz und koronare Interventionen, wie beispielsweise Angioplastie, Stenting oder Atherektomie, Hyperplasie nach Angioplastie, Atherektomie oder arteriellem Stenting, Verschlusssyndrom in einem Gefäßsystem oder fehlender Durchgängigkeit erkrankter Arterien (lack of patency of diseased arteries), thrombotischer thrombozytopenischer Purpura (TTP), transitorischer ischämischer Hirnattacke, instabiler oder stabiler Angina pectoris, Hirninfarkt, HELLP-Syndrom, Karotis-Endarterektomie, Verengung der Halsschlagader, kritischer Ischämie der Gliedmaßen (critical limb ischaemia), kardialer Embolie (cardioembolism), peripherer Gefäßerkrankung, Restenose und/oder Myokardinfarkt.

18. Verwendung gemäß Anspruch 13, wobei der Nanokörper, Domänen-Antikörper, Einzeldomänen-Antikörper oder dAb gegen IL-6, IL-6R und/oder IL-6/IL-6R-Komplex gerichtet ist, für die Herstellung eines Medikaments zur Prävention und/oder Behandlung der Bildung von Sepsis, verschiedener Formen von Krebs, wie beispielsweise Multiplem Myelom (MM), Nierenzellkarzinom (renal cell carcinoma; RCC), Plasmazell-Leukämie, Lymphom, B-lymphoproliferativer Störung (B-lymphoproliferative disorder; BLPD) und Prostatakrebs, Knochenresorption (Osteoporose), Kachexie, Psoriasis, mesangialer proliferativer Glomerulonephritis, Kaposi-Sarkom, AIDS-verwandtem Lymphom, inflammatorischer Erkrankungen und Störungen, wie beispielsweise rheumatoider Arthritis, systemischer juveniler idiopathischer Arthritis, Hypergammaglobulinämie, Morbus Crohn, ulzerativer Colitis, systemischem Lupus erythematodes (SLE), Multipler Sklerose, Castleman-Erkrankung, IgM-Gammopathie, kardialem Myxom, Asthma (insbesondere allergischem Asthma) und/oder autoimmunem insulinpflichtigem Diabetes mellitus.

## Revendications

1. Composition qui comprend une quantité thérapeutiquement efficace
(i) d'un polypeptide, ledit polypeptide étant constitué d'un ou de deux Nanobodies, anticorps à domaine, anticorps à domaine unique ou dAb, facultativement liés par l'intermédiaire d'un lieur,
(ii) et d'un support nasal pharmaceutiquement acceptable,
pour son utilisation dans un procédé de traitement comprenant l'induction d'une distribution systémique du polypeptide par administration intranasale aux muqueuses nasales d'un sujet.

2. Composition pour son utilisation selon la revendication 1, dans laquelle ledit Nanobody, anticorps à domaine, anticorps à domaine unique ou dAb est dirigé contre une cible dans le rein ou la vessie, pour la prévention et/ou le traitement d'une maladie ou d'un trouble du rein ou de la vessie.

3. Composition pour son utilisation selon la revendication 1, dans laquelle ledit Nanobody, anticorps à domaine, anticorps à domaine unique ou dAb est dirigé contre une cible sur une tumeur, pour la prévention et/ou le traitement d'au moins une maladie ou un trouble associé à une tumeur.

4. Composition pour son utilisation selon la revendication 1, dans laquelle ledit Nanobody, anticorps à domaine, anticorps à domaine unique ou dAb est dirigé contre le TNF, pour la prévention et/ou le traitement d'au moins une maladie ou un trouble tel qu'une maladie auto-immune, dans laquelle la maladie auto-immune est facultativement une polyarthrite rhumatoïde ou une affection abdominale inflammatoire.

5. Composition pour son utilisation selon la revendication 1, dans laquelle ledit Nanobody, anticorps à domaine, anticorps à domaine unique ou dAb est dirigé contre le vWF pour son utilisation dans un procédé de prévention et/ou de traitement de la formation d'un thrombus non occlusif, de la formation d'un thrombus occlusif, de la formation d'un thrombus artériel, d'une occlusion coronarienne aiguë, d'une maladie occlusive des artères périphériques, d'une resténose et des troubles résultant d'un pontage coronarien, du remplacement d'une valvule d'artère coronarienne et d'interventions coronariennes telles qu'une angioplastie, la pose d'un stent ou une athérectomie, d'une hyperplasie après une angioplastie, une athérectomie ou la pose d'un stent artériel, d'un syndrome occlusif dans un système vasculaire ou d'un manque de perméabilité d'artères malades, du purpura thrombocytopénique thrombotique (TTP), d'une attaque ischémique cérébrale transitoire, d'une angine de poitrine instable ou stable, d'un infarctus cérébral, d'un syndrome HELLP, d'une endartériectomie carotidienne, d'une sténose d'artère carotidienne, d'une ischémie critique d'un membre, d'une embolie cardiaque, d'une maladie vasculaire périphérique, d'une resténose et/ou d'un infarctus du myocarde.

6. Composition pour son utilisation selon la revendication 1, dans laquelle ledit Nanobody, anticorps à domaine, anticorps à domaine unique ou dAb est dirigé contre l'IL-6, l'IL-6R et/ou le complexe IL-6/IL-6R pour son utilisation dans un procédé de prévention et/ou de traitement de la formation d'une septicémie, de diverses formes de cancer tel qu'une maladie de type myélome multiple (MM), un carcinome à cellules rénales (RCC), une leucémie à cellules plasmatiques, un lymphome, un trouble lymphoprolifératif B (BLPD) et un cancer de la prostate, une résorption osseuse (ostéoporose), une cachexie, un psoriasis, une glomérulonéphrite proliférative mésangiale, un sarcome de Kaposi, un lymphome associé au sida, des maladies et troubles inflammatoires tels que la polyarthrite rhumatoïde, l'arthrite idiopathique juvénile systémique, l'hypergammaglobulinémie, la maladie de Crohn, la rectocolite hémorragique, le lupus érythémateux disséminé (SLE), la sclérose en plaques, la maladie de Castleman, la gammapathie à IgM, le myxome cardiaque, l'asthme, en particulier l'asthme allergique et/ou le diabète sucré insulinodépendant auto-immun.

7. Composition pour son utilisation selon l'une quelconque des revendications 1 à 6, dans laquelle ledit Nanobody, anticorps à domaine, anticorps à domaine unique ou dAb est un V_{HH} humanisé ou un V_{H} camélisé.

8. Composition pour son utilisation selon l'une quelconque des revendications 1 à 7, dans laquelle le support nasal est liquide, semi-liquide, un gel, une crème, une pâte, une poudre ou un solide, ou peut être un film ou un matériau d'encapsulation.

9. Composition pour son utilisation selon l'une quelconque des revendications 1 à 8, comprenant en outre un ou plusieurs ingrédients thérapeutiques supplémentaires.

10. Composition pour son utilisation selon l'une quelconque des revendications 1 à 9, qui est comprise dans un dispositif de distribution pour administration intranasale, qui est pourvu d'un moyen permettant l'application de ladite composition sur la muqueuse nasale.

11. Composition pour son utilisation selon la revendication 10, dans laquelle ledit moyen permettant l'application de ladite composition sur la muqueuse nasale est sélectionné dans le groupe des dispositifs de distribution consistant en :
- un récipient pourvu d'un dispositif à pipette/fermeture classique ;
- une pulvérisation à pompe à flacon souple ;
- une pulvérisation sans air et sans conservateur ;
- un insert nasal.

12. Composition pour son utilisation selon la revendication 11, dans laquelle ledit dispositif de distribution est un système doseur.

13. Utilisation
(i) d'un polypeptide, ledit polypeptide étant constitué d'un ou de deux Nanobodies, anticorps à domaine, anticorps à domaine unique ou dAb, facultativement liés par l'intermédiaire d'un lieur,
(ii) et d'un support nasal pharmaceutiquement acceptable,
pour la préparation d'un médicament qui comprend une quantité thérapeutiquement efficace du polypeptide, pour son utilisation dans un procédé de traitement comprenant la distribution systémique du polypeptide par administration intranasale aux muqueuses nasales d'un sujet.

14. Utilisation selon la revendication 13, dans laquelle ledit Nanobody, anticorps à domaine, anticorps à domaine unique ou dAb est dirigé contre une cible dans le rein ou la vessie, pour la préparation d'un médicament pour la prévention et/ou le traitement d'une maladie ou d'un trouble du rein ou de la vessie.

15. Utilisation selon la revendication 13, dans laquelle ledit Nanobody, anticorps à domaine, anticorps à domaine unique ou dAb est dirigé contre une cible sur une tumeur, pour la préparation d'un médicament pour la prévention et/ou le traitement d'au moins une maladie ou un trouble associé à une tumeur.

16. Utilisation selon la revendication 13, dans laquelle ledit Nanobody, anticorps à domaine, anticorps à domaine unique ou dAb est dirigé contre le TNF, pour la préparation d'un médicament pour la prévention et/ou le traitement d'au moins une maladie ou un trouble tel qu'une maladie auto-immune, dans laquelle la maladie auto-immune est facultativement une polyarthrite rhumatoïde ou une affection abdominale inflammatoire.

17. Utilisation selon la revendication 13, dans laquelle ledit Nanobody, anticorps à domaine, anticorps à domaine unique ou dAb est dirigé contre le vWF, pour la préparation d'un médicament pour la prévention et/ou le traitement de la formation d'un thrombus non occlusif, de la formation d'un thrombus occlusif, de la formation d'un thrombus artériel, d'une occlusion coronarienne aiguë, d'une maladie occlusive des artères périphériques, d'une resténose et des troubles résultant d'un pontage coronarien, du remplacement d'une valvule d'artère coronarienne et d'interventions coronariennes telles qu'une angioplastie, la pose d'un stent ou une athérectomie, d'une hyperplasie après une angioplastie, une athérectomie ou la pose d'un stent artériel, d'un syndrome occlusif dans un système vasculaire ou d'un manque de perméabilité d'artères malades, du purpura thrombocytopénique thrombotique (TTP), d'une attaque ischémique cérébrale transitoire, d'une angine de poitrine instable ou stable, d'un infarctus cérébral, d'un syndrome HELLP, d'une endartériectomie carotidienne, d'une sténose d'artère carotidienne, d'une ischémie critique d'un membre, d'une embolie cardiaque, d'une maladie vasculaire périphérique, d'une resténose et/ou d'un infarctus du myocarde.

18. Utilisation selon la revendication 13, dans laquelle ledit Nanobody, anticorps à domaine, anticorps à domaine unique ou dAb est dirigé contre l'IL-6, l'IL-6R et/ou le complexe IL-6/IL-6R, pour la préparation d'un médicament pour la prévention et/ou le traitement de la formation d'une septicémie, de diverses formes de cancer tel qu'une maladie de type myélome multiple (MM), un carcinome à cellules rénales (RCC), une leucémie à cellules plasmatiques, un lymphome, un trouble lymphoprolifératif B (BLPD), et un cancer de la prostate, une résorption osseuse (ostéoporose), une cachexie, un psoriasis, une glomérulonéphrite proliférative mésangiale, un sarcome de Kaposi, un lymphome associé au sida, des maladies et troubles inflammatoires tels que la polyarthrite rhumatoïde, l'arthrite idiopathique juvénile systémique, l'hypergammaglobulinémie, la maladie de Crohn, la rectocolite hémorragique, le lupus érythémateux disséminé (SLE), la sclérose en plaques, la maladie de Castleman, la gammapathie à IgM, le myxome cardiaque, l'asthme (en particulier l'asthme allergique) et/ou le diabète sucré insulinodépendant auto-immun.
